# EUROPÄISCHE PATENTSCHRIFT

(11) **EP 0 812 844 B1**
(45) Veröffentlichungstag und Bekanntmachung des Hinweises auf die Patenterteilung: **23.10.2002**
(21) Anmeldenummer: 97108870.3
(22) Anmeldetag: 03.06.1997
(51) Int. Cl.: C07D 473/04, A61K 31/52

(54) **Verwendung von Theophyllinderivaten zur Behandlung und Propylaxe von Schockzuständen, neue Xanthinverbindungen und Verfahren zu deren Herstellung**
Use of theophyllin derivatives for the treatment and prophylaxis fo shock conditions, novel xanthine compounds and processes for the production thereof
Utilisation de dérivés de théophylline pour la prophylaxie et le traitement des conditions de choc, nouveaux composés à base de xanthine et leurs procédés de préparation

(30) Priorität: 07.06.1996 DE 19622737; 24.07.1996 DE 19629815
(43) Veröffentlichungstag der Anmeldung: 17.12.1997
(73) Patentinhaber: HOECHST AKTIENGESELLSCHAFT, 65929 Frankfurt am Main (DE)
(72) Erfinder: Gebert, Ulrich, Dr., 61479 Glashütten (DE); Wolf, Erhard, Dr., 65719 Hofheim (DE); Defossa, Elisabeth, Dr., 65510 Idstein (DE); Heinelt, Uwe, Dr., 65187 Wiesbaden (DE); Anagnostopulos, Hiristo, DB., 65193 Wiesbaden (DE); Rudolphi, Karl, Dr., 55116 Mainz (DE); Grome, John J., Dr., 65207 Wiesbaden (DE)

(56) Entgegenhaltungen:
- EP-A- 0 462 506
- EP-A- 0 771 813
- US-A- 4 904 472
- P. MASOUYE ET AL.: "Yanthine Derivative HWA 138 Attenuates Hemodynamic and Oxygen Uptake Dysfunction Secondary to Severe Endotoxin Shock in Sheep" CIRC. SHOCK, Bd. 36, Nr. 2, 1992, Seiten 113-119, XP002054948
- K. L. BERENS ET AL.: "Influence of Pentoxifylline and Related Analogues in Endotoxemic Renal Failure" CIRC. SHOCK, Bd. 34, Nr. 3, 1991, Seiten 344-348, XP002054949
- J. P. TARAYRE ET AL.: "Theophylline reduces pulmonary eosinophilia after various types of active anaphylactic shock in guinea-pigs" J. PHARM. PHARMACOL., Bd. 43, Nr. 12, 1991, Seiten 877-879, XP002054950
- B. EICHELMAN ET AL.: "Methylxanthine-Facilitated Shock-Induced Aggression in the Rat" PSYCHOPHARMACOLOGY (BERLIN), Bd. 56, Nr. 3, 1978, Seiten 305-308, XP002054951
- E. OKABE: "Preventive effects of theophylline on anaphylactic shock in rats" JPN. J. PHARMACOL., Bd. 30, Nr. 3, 1980, Seiten 367-376, XP002054952
- I. MARZI ET AL.: "Influence of Pentoxifylline and Albifylline on Liver Microcirculation and Leukocyte Adhesion after Hemorrhagic Shock in the Rat" J. TRAUMA: INJ., INFECT., CRIT. CARE, Bd. 40, Nr. 1, 1996, Seiten 90-96, XP002054953
- PATENT ABSTRACTS OF JAPAN vol. 015, no. 403, 15.Oktober 1991 & JP 03 167186 A (HOKURIKU SEIYAKU CO., LTD), 19.Juli 1991,

## Beschreibung

Die vorliegende Erfindung betrifft die Verwendung von Theophyllinderivaten mit mindestens einer Etherfunktion im strukturell modifizierten 1-ständigen Methylrest zur Herstellung von Arzneimitteln für die Behandlung und Prophylaxe von Schockerkrankungen, neue Xanthinverbindungen mit vorgenanntem Substitutionsmuster und Verfahren zu deren Herstellung.

Schock wird als ein akut auftretender Zustand inadäquater nutritiver Perfusion lebenswichtiger Organe definiert, der stets höchste Lebensgefahr bedeutet (Med. Mo. Pharm. 1989, 12/9: 279 - 282).

Die Schockursachen sind vielfältig. So wird der kardiogene Schock durch primäres Herzversagen infolge von Myokardinfarkt, schweren Herzrhythmusstörungen, Herzmuskelinsuffizienz oder anderen Herzerkrankungen, der hypovolämische Schock (hämorrhagischer und traumatischer Schock sowie Verbrennungs- und Dehydratationsschock) durch Flüssigkeitsverluste oder -verschiebungen, der septische Schock durch systemische Einschwemmung von Mikroben (gramnegative und grampositive Bakterien, Pilze, Viren, Protozoen etc.) oder deren Toxinen und schließlich der anaphylaktische Schock durch generalisierte Antigen-Antikörper-Reaktionen ausgelöst.Trotz dieser Ursachenvielfalt erweisen sich jedoch Pathogenese und klinisches Bild der verschiedenen Schockformen als recht einheitlich (Pschyrembel, Klinisches Wörterbuch, Walter de Gruyter-Verlag, 255. Auflage, 1986, Seite 1513). Schlüsselrolle spielt immer eine Störung der Zellfunktionen infolge unzureichender Versorgung der Gewebe mit Sauerstoff und Substraten (Ischämie) und mangelhafter Entsorgung der toxischen Stoffwechselprodukte (Medwelt 1989, 40: 519 - 522). Schock ist ein dynamisches Geschehen, dessen Verlauf maßgeblich von der Ischämiedauer abhängt.
In der ersten, kompensierten Schockphase reagiert der Organismus mit einer neuronal und hormonal gesteuerten Zentralisation des Kreislaufs, durch die im Körperzentrum gelegene Organe (Herz, Gehirn, Lunge, Leber, Nieren) vorerst geschützt werden. Das klinische Bild wird bestimmt durch Tachykardie, noch normalen oder nur geringfügig erniedrigten Blutdruck, Hyperventilation mit respiratorischer Alkalose und in der Regel blasse, kalte und feuchte Haut; beim septischen Schock tritt auch Fieber, bisweilen verbunden mit Schüttelfrost, auf. Sind die Kompensationsmechanismen ausgeschöpft, so wird in zunehmendem Maße auch die kapillare Perfusion der zentralen Organe beeinträchtigt. Dies leitet in die zweite, dekompensierte Schockphase über, die durch fortschreitenden Zelluntergang und Funktionsverlust gekennzeichnet ist. Das Schockgeschehen wird irreversibel. Die drastische Erhöhung der Gefäßpermeabilität im Mikrozirkulationsbereich führt durch Flüssigkeitsverlust zum Hämatokritanstieg, zu interstitiellen Ödemen und zur Freisetzung von Mediatoren, die unter anderem eine disseminierte intravasale Gerinnung, etwa in Form einer Verbrauchskoagulopathie mit obturierenden Fibrinthromben in der terminalen Strombahn, auslösen. Die stetige Reduktion von Herzzeitvolumen und Blutdruck leitet zum völligen Kreislaufzusammenbruch über. Am Ende der Schockkaskade steht der Tod durch akutes Versagen von Herz, Leber, Nieren oder Lunge (akutes Atemnotsyndrom, auch ARDS = Acute Respiratory Distress Syndrome genannt) oder durch Multiorganversagen (MOF = Multi-Organ Failure), wenn mehrere Organe gleichzeitig ihre Funktion verlieren.

Die konventionelle Therapie orientiert sich an der klinischen Symptomatik und umfaßt Sofortmaßnahmen zur Beseitigung der Vitalbedrohung, wie Volumensubstitution, künstliche Beatmung zur Prophylaxe eines ARDS, Verabreichung vasoaktiver Pharmaka zur Stützung des Kreislaufs, Analgesie und Sedierung, Korrektur der Störungen im Säure-Basen-Haushalt, Heparingabe zur Vermeidung einer Verbrauchskoagulopathie und Behandlung mit Kortikosteroiden zur Verringerung der Membranpermeabilität. Je nach Schockursache sind weitere Therapiemaßnahmen indiziert, beispielsweise Operation und Blutstillung beim hämorrhagischen Schock, Elimination des Infektionsherdes und Antibiotikatherapie beim septischen Schock und eventuelle Behandlung mittels Herzschrittmacher und aortale Ballongegenpulsation beim kardiogenen Schock. Ungeachtet all dieser therapeutischen Maßnahmen bleibt das Behandlungsergebnis jedoch außerordentlich unbefriedigend. So beträgt die Mortalitätsrate beispielsweise beim kardiogenen Schock aufgrund eines Herzinfarkts 90% und beim septischen Schock, der weltweit häufigsten Todesursache auf Intensivstationen, mehr als 50%.

Dies macht die Forderung von Klinikern nach einem mehr kausal ausgerichteten Therapiekonzept verständlich, das eine möglichst frühzeitige Unterbrechung der Schockkaskade gestattet und damit die Überlebenschance deutlich verbessert. Erfolgversprechende Ansätze hierfür bieten die komplexen pathophysiologischen Prozesse, die dem progressiven Verlauf der Schockerkrankung zugrunde liegen. Nach gegenwärtigem Kenntnisstand wird sowohl bei septischen als auch aseptischen Schockformen (N. Engl. J. Med. 1993, 328/20: 1471 - 1477) durch den jeweiligen pathologischen Stimulus eine Vielzahl von Mediatorsystemen und inflammatorisch kompetenten Zellen aktiviert und dadurch eine endotheliale Entzündung mit diffusen entzündlichen Prozessen ausgelöst, die man auch als SIRS (Systemic Inflammatory Response Syndrome) bezeichnet (J. Amer. med. Ass. 1992, 268: 3452). Im Mittelpunkt dieses Syndroms steht die generalisierte pathologische Interaktion zwischen aktivierten Granulozyten und Endothelzellen über komplementäre Adhäsionsmoleküle, die unter fortschreitender Gefäßschädigung zu Störungen in der Mikrozirkulation und Organschäden mit zunehmender Funktionsbeeinträchtigung führt und schließlich in ein Multiorganversagen einmündet. Mit Auslösung der Gefäßwand-assoziierten inflammatorischen Prozesse durch die granulozytärendotheliale Interaktion folgen septisches und aseptisches Geschehen einer gemeinsamen pathogenetischen Endstrecke bei der Schockentwicklung. Darüber hinaus gibt es stichhaltige Hinweise dafür, daß es im Verlaufe aseptischer Schockformen sehr häufig über eine initial nicht mikrobiell getriggerte Schrankenstörung in Lunge und insbesondere Gastrointestinaltrakt zu einer als bakterielle Translo-kation bezeichneten Invasion von Bakterien oder deren toxischen Produkten in die Blutbahn kommt, so daß sich aseptisches und septisches Geschehen überlagern (Medwelt 1989, 40: 525 - 532).

Neuere Versuche zu einer kausalen therapeutischen Intervention zielen nun auf spezifische Eingriffe in den durch Entzündungsmediatoren unterhaltenen Krankheitsprozeß ab, um die pathologische Signalkette so frühzeitig wie möglich zu unterbrechen und damit der Entwicklung von Organschäden rechtzeitig vorzubeugen. In großangelegten klinischen Studien sind beispielsweise murine und humane monoklonale Antikörper gegen das Endotoxin (LPS = Lipopolysaccharide) aus der Zellwand gramnegativer Bakterien, humanisierte rekombinante und sowohl murine als auch humane monoklonale Antikörper gegen das Zytokin TNF (Tumornekrosefaktor), gentechnologisch hergestellte lösliche TNF-Rezeptoren und andere TNFbindende Proteine, der durch Rekombination gewonnene, physiologisch auftretende Interleukin-1-Rezeptor-Antagonist Antril (IL-1-RA) sowie der Bradykinin-Antagonist Bradycor untersucht worden, ohne daß sich bislang ein therapeutischer Durchbruch abzeichnet (Scrip Magazine, December 1994: 50 - 52). Die intensive Suche nach effektiven Blockern des außerordentlich komplexen Krankheitsgeschehens geht daher unvermindert weiter, wobei sich zunehmend die Erkenntnis durchsetzt, daß die Ausschaltung eines spezifischen Mediators der breitgefächerten Signalkaskade nur geringe Erfolgsaussichten hat und daß Therapiefortschritte am ehesten von einem multifunktionellen Eingriff zu erwarten sind, sei es nun durch Kombination verschiedener selektiv wirkender Pharmaka oder vorteilhafter durch ein Monopharmakon mit möglichst breitem pharmakologischem Wirkungsspektrum.

Für die Prüfung von Präparaten auf Antischockwirkung sind verschiedene experimentelle Tiermodelle entwickelt worden. Ein besonders praktikables, gut standardisiertes und aussagekräftiges Modell (Proc. Natl. Acad. Sci. USA 1979, 76/11: 5939 - 5943) stellt der mit Endotoxin (LPS) induzierte Schock an C57BL/6-Mäusen dar, das die klinische Situation insofern realistisch nachstellt, als durch gleichzeitige Gabe von Galaktosamin (GaIN) die Empfindlichkeit der Tiere gegenüber LPS so stark erhöht wird, daß die verhältnismäßig niedrige Letaldosis beim Menschen auch hier für die Auslösung des tödlichen Schockgeschehens ausreicht (DN&P 1993, 6/9: 641 - 646; Biospektrum 1995, 1/5: 46 - 52). In diesem Modell zeigt Theophyllin (1,3-Dimethylxanthin) in Dosen bis zur Verträglichkeitsgrenze keine nennenswerte Schutzwirkung.

Überraschend wurde nun gefunden, daß die Einführung von Substituenten mit mindestens einer Etherfunktion in den 1-ständigen Methylrest des Theophyllinmoleküls sehr potente Präparate bei gleichzeitig wesentlich verbesserter Verträglichkeit liefert. Es sind drei Verbindungen dieses Strukturtyps bekannt, und zwar die 3-n-Propylxanthine mit der 2-Methoxyethyl-, 2-Ethoxyethyl- oder 3-Methoxypropyl-Gruppe in 1-Position (J. Med. Chem. 1993, 36/10: 1380 - 1386), die sich aufgrund bronchodilatatorischer Eigenschaften zur Behandlung akuter Asthmabeschwerden eignen sollen, aber Hinweise auf ihre Verwendbarkeit als Antischockmittel existieren nicht.

Die Erfindung betrifft die Verwendung von mindestens einer Verbindung der Formel I, 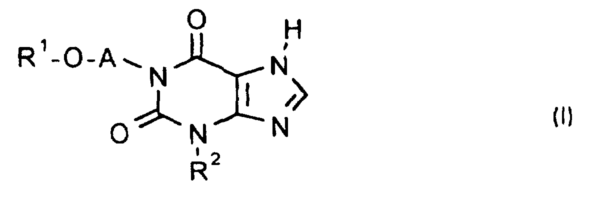 wobei
- R¹: für
a) geradkettiges oder verzweigtes (C₁-C₅)-Alkyl,
b) (C₁-C₂)-Alkoxy-(C₁-C₃)-alkyl oder
c) Phenyl oder Phenyl-(C₁-C₂)-alkyl steht, worin die Phenylreste unsubstituiert oder jeweils mit einem oder zwei Halogenatomen substituiert sind,
- A: für eine unverzweigte oder verzweigte (C₁-C₄)-Alkylenbrücke steht und
- R²: für
a) geradkettiges oder verzweigtes (C₁-C₅)-Alkyl,
b) (C₃-C₆)-Cycloalkyl,
c) (C₄-C₈)-Cycloalkyl-alkyl,
d) Phenyl oder
e) Phenyl-(C₁-C₂)-alkyl steht,
zur Herstellung eines Arzneimittels für die Behandlung und Prophylaxe von Schockerkrankungen, insbesondere von SIRS (Systemic Inflammatory Response Syndrome), Sepsis, Sepsis-Syndrom, septischem Schock, Multiorganversagen (MOF), ARDS (Acute Respiratory Distress Syndrome), hämorrhagischem und traumatischem Schock sowie Verbrennungs- und Dehydratationsschock und schockähnlichen Komplikationen beim Reperfusionssyndrom und extrakorporalen Kreislauf.

Bevorzugt werden Verbindungen der Formel I eingesetzt, wobei
- R¹: für
a) geradkettiges oder verzweigtes (C₁-C₄)-Alkyl,
b) Methoxymethyl,
c) Methoxyethyl,
d) Phenyl,
e) 4-Chlorphenyl,
f) Benzyl oder
g) 4-Chlorbenzyl steht,
- A: für eine unverzweigte (C₁-C₃)-Alkylenbrücke steht und
- R²: für
a) geradkettiges oder verzweigtes (C₁-C₄)-Alkyl,
b) Cyclopropyl,
c) Cyclopropylmethyl,
d) Phenyl oder
e) Benzyl steht.

Weiterhin bevorzugt ist der Einsatz der Verbindungen der Formel I, wobei
- R¹: geradkettiges oder verzweigtes (C₁-C₄)-Alkyl,
- A: eine unverzweigte (C₁-C₃)-Alkylenbrücke und
- R²: geradkettiges oder verzweigtes (C₁-C₄)-Alkyl, Cyclopropyl oder Cyclopropylmethyl bedeuten.

Der Ausdruck "(C₄-C₈)-Cycloalkyl-alkyl" definiert solche Alkylreste, die mit (C₃-C₆)-Cycloalkyl substituiert sind, wobei die Summe aller C-Atome kleiner oder gleich 8 ist. Dies sind der Cyclopropyl-methyl bis -pentyl-, Cyclobutylmethyl- bis -butyl-, Cyclopentyl-methyl- bis -propyl- sowie Cyclohexyl-methylund -ethyl-Rest. Halogenatome bedeuten Jod, Brom, Fluor und vorzugsweise Chlor.

Die Erfindung betrifft auch neue Verbindungen der Formel I, in der
- R¹: für
a) geradkettiges oder verzweigtes (C₁-C₅)-Alkyl,
b) (C₁-C₂)-Alkoxy-(C₁-C₃)-alkyl oder
c) Phenyl oder Phenyl-(C₁-C₂)-alkyl steht, worin die Phenylreste unsubstituiert oder jeweils mit einem oder zwei Halogenatomen substituiert sind,
- A: für eine unverzweigte oder verzweigte (C₁-C₄)-Alkylenbrücke steht und
- R²: für
a) geradkettiges oder verzweigtes (C₁-C₅)-Alkyl,
b) (C₃-C₆)-Cycloalkyl,
c) (C₄-C₈)-Cycloalkyl-alkyl,
d) Phenyl oder
e) Phenyl-(C₁-C₂)-alkyl steht,
wobei Verbindungen der Formel I, worin a) R² für n-Propyl, R¹ für Methyl oder Ethyl und A für eine Ethylenbrücke oder b) R² für n-Propyl, R¹ für Methyl und A für eine n-Propylenbrücke stehen, ausgenommen sind.

Bevorzugt sind Verbindungen der Formel I, in der
- R¹: für
a) geradkettiges oder verzweigtes (C₁-C₄)-Alkyl,
b) Methoxymethyl,
c) Methoxyethyl,
d) Phenyl,
e) 4-Chlorphenyl,
f) Benzyl oder
g) 4-Chlorbenzyl steht,
- A: für eine unverzweigte (C₁-C₃)-Alkylenbrücke steht und
- R²: für
a) geradkettiges oder verzweigtes (C₁-C₄)-Alkyl,
b) Cyclopropyl,
c) Cyclopropylmethyl,
d) Phenyl oder
e) Benzyl steht,
wobei Verbindungen der Formel I, worin a) R² für n-Propyl, R¹ Methyl oder Ethyl und A für eine Ethylenbrücke oder b) R² für n-Propyl, R¹ für Methyl und A für eine n-Propylenbrücke stehen, ausgenommen sind.

Weiterhin bevorzugt sind Verbindungen der Formel I, in der
- R¹: geradkettiges oder verzweigtes (C₁-C₄)-Alkyl,
- A: eine unverzweigte (C₁-C₃)-Alkylenbrücke und
- R²: geradkettiges oder verzweigtes (C₁-C₄)-Alkyl, Cyclopropyl oder Cyclopropylmethyl bedeuten,
wobei die Verbindungen der Formel I, worin a) R² für n-Propyl, R¹ für Methyl oder Ethyl und A für eine Ethylenbrücke oder b) R² für n-Propyl, R¹ für Methyl und A für eine n-Propylenbrücke stehen, ausgenommen sind.

Die Verbindungen der Formel I lassen sich in der 7-Position deprotonieren und bilden daher mit basischen Agentien Salze und Solvate. Hierfür kommen vorzugsweise die pharmazeutisch akzeptablen Alkali- und Erdalkalimetallsalze und die Salze und Solvate mit organischen Basen, beispielsweise Ethylendiamin oder die basischen Aminosäuren Lysin, Ornithin und Arginin, in Frage. Die Erfindung betrifft somit auch die physiologisch verträglichen Salze und/oder Solvate der 1,3-disubstituierten Xanthine gemäß Formel I und deren Verwendung als Wirkstoffe in Antischockmitteln.

Verbindungen der Formel I mit unsymmetrisch verzweigtem Alkylrest in der Position von R¹ und/oder R² und/oder mit einer unsymmetrisch verzweigten Alkylenbrücke A besitzen ein oder mehrere asymmetrische C-Atome und können somit in stereoisomeren Formen vorliegen. Die Erfindung schließt daher sowohl die stereoisomerenreinen Verbindungen als auch deren Gemische und deren Verwendung als Wirkstoffe in Antischockmitteln mit ein.

Die Erfindung betrifft ferner ein Analogieverfahren zur Herstellung der neuen Verbindungen gemäß Formel I, dessen prinzipielle Ausführungsformen in der WO 87/00523 beschrieben sind. Beispielsweise wird so vorgegangen, daß man
a) ein 3-substituiertes Xanthin der Formel II, 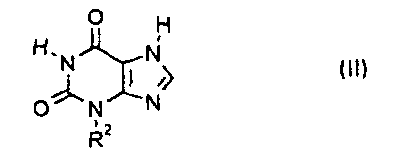 worin R² wie in Formel I definiert ist, ohne Kondensationsmittel oder in Gegenwart eines basischen Kondensationsmittels oder in Form seiner Salze mit einem Reagens der Formel III,

   R^{a}-X (III)

   worin R^{a} eine leicht eliminierbare Abgangsgruppe, beispielsweise die reduktiv oder auch hydrolytisch entfernbare Benzyl-, Benzhydryl- oder Tritylgruppe mit unsubstituierten oder substituierten Phenylringen, und X Halogen, vorzugsweise Chlor, Brom oder Jod, oder alternativ eine Sulfonsäureester- oder Phosphorsäureester-Gruppierung bedeuten, oder
b) ein 7-substituiertes Xanthin der Formel IV, 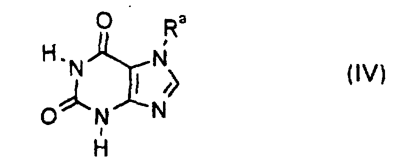 worin R^{a} Benzyl mit unsubstituiertem oder substituiertem Phenylrest bedeutet, ohne Kondensationsmittel oder in Gegenwart eines basischen Kondensationsmittels oder in Form seiner Salze mit einem Reagens der Formel V,

   R²-X (V)

   worin R² wie in Formel I und X wie in Formel III definiert sind,
zu einem 3,7-disubstituierten Xanthin der Formel VI 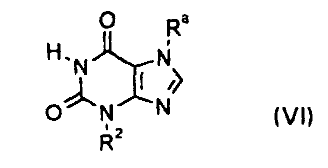 umsetzt, worin R² wie in Formel I und R^{a} wie in Formel III oder IV definiert sind, anschließend die Verbindung der Formel VI ohne Kondensationsmittel oder in Gegenwart eines basischen Kondensationsmittels oder in Form seiner Salze mit einem Alkylierungsmittel der Formel VII,

R¹-O-A-X (VII)

worin R¹ und A wie in Formel I und X wie in Formel III definiert sind,
in ein 1,3,7-trisubstituiertes Xanthin der Formel VIII 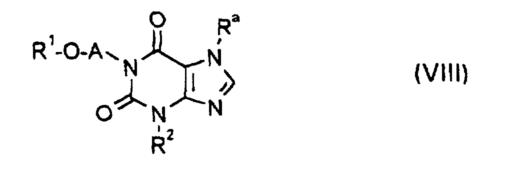 umwandelt, worin R¹, A und R² wie in Formel I und R^{a} wie in Formel III oder IV definiert sind,
und abschließend durch Eliminierung der Abgangsgruppe R^{a} aus dem Zwischenprodukt der Formel VIII die erfindungsgemäße Verbindung der Formel I gewinnt und diese, gegebenenfalls nach Trennung der stereoisomeren Formen wahlfrei in ein physiologisch verträgliches Salz überführt.

Die hierbei als Ausgangsstoffe verwendeten monosubstituierten Xanthine der Formeln II und IV und Alkylierungsmittel der Formeln III, V und VII sind größtenteils bekannt oder lassen sich nach bekannten Methoden leicht herstellen.
So sind beispielsweise die 7-Benzylxanthine der Formel IV aus Guanosin durch Benzylierung, hydrolytische Eliminierung des Zuckerrestes und anschließende Umwandlung des Guanosin- in das Xanthingerüst zugänglich (Synth. Commun. 1990, 20: 2459 - 2467).
Unter den zur Einführung der R¹-O-A-Seitenkette in die 1-Position des Xanthingerüstes geeigneten Alkylierungsmitteln der Formel VII nehmen jene Verbindungen, in denen A eine Methylen-Gruppe (A = -CH₂-) bedeutet, insofern eine Sonderstellung ein, als deren Halogenide zwar erfolgreich als Reaktanten einsetzbar sind, aber zumindest bei großtechnischer Anwendung toxikologische Probleme aufwerfen können. Daher kann in diesem speziellen Fall der Einsatz der entsprechenden Sulfonate bevorzugt sein, die beispielsweise durch Umsetzung von gemischten Anhydriden aliphatischer Carbonsäuren und aliphatischer oder aromatischer Sulfonsäuren (J. Org. Chem. 1971, 36: 528 - 531) mit den disubstituierten Formaldehydacetalen der Formel IX in übersichtlich und nahezu vollständig verlaufender Reaktion bequem zugänglich sind (J. Amer. Chem. Soc. 1969, 91: 5663 - 5665):

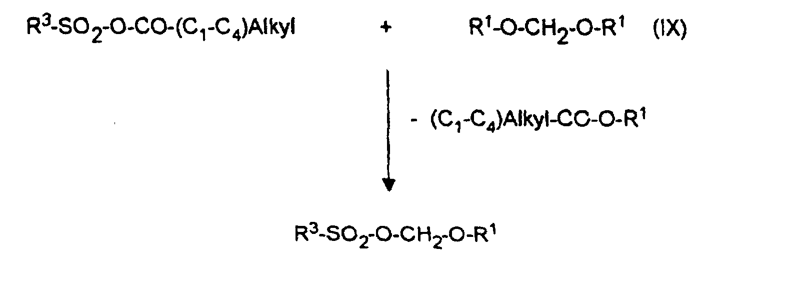

Hierbei stellt R³ einen aliphatischen Rest wie Methyl, Ethyl oder Trifluormethyl oder einen aromatischen Rest, beispielsweise Phenyl, 4-Tolyl oder 4-Bromphenyl, vorzugsweise aber Methyl oder 4-Tolyl, dar und R¹ hat die bei Formel I definierten Bedeutungen.
Die Reaktion kann sowohl in Substanz als auch in einem wasserfreien, gegenüber den Reaktionspartnern inerten aprotischen Lösungsmittel bei Temperaturen zwischen -20° und +40°C, vorzugsweise zwischen 0° und 20°C, durchgeführt werden. Eine Zwischenisolierung der hochreaktiven, hydrolyseempfindlichen und hitzelabilen Sulfonate ist nicht erforderlich; sie werden zweckmäßig unmittelbar als Rohprodukte zur Alkylierung der Xanthine VI am Stickstoffatom in 1-Position verwendet, wobei sich häufig der sonst übliche Zusatz eines basischen Kondensationsmittels erübrigt.

Die Umsetzung der mono- und disubstituierten Xanthinderivate II, IV und VI mit den betreffenden Alkylierungsmitteln der Formel III, V oder VII erfolgt gewöhnlich in einem gegenüber den Reaktionsteilnehmern inerten Verteilungsoder Lösungsmittel. Als solche kommen vor allem dipolare, aprotische Solventien, beispielsweise Dimethylformamid, Dimethylacetamid, N-Methylpyrrolidon, Tetramethylharnstoff, Hexamethylphosphorsäuretriamid oder Dimethylsulfoxid, in Frage; es können aber auch Formamid, Acetonitril, Aceton, Butanon oder Alkohole, wie Methanol, Ethylenglykol und dessen Mono- bzw. Di(C₁-C₄)alkylether, Ethanol, Propanol, Isopropanol und die verschiedenen Butanole; Kohlenwasserstoffe, wie Benzol, Toluol oder Xylol; halogenierte Kohlenwasserstoffe, wie Dichlormethan oder Chloroform; Pyridin sowie Mischungen der genannten Lösungsmittel oder deren Gemische mit Wasser Verwendung finden. Die Alkylierungsreaktionen werden zweckmäßig in Gegenwart eines basischen Kondensationsmittels durchgeführt. Hierfür eignen sich beispielsweise Alkali- oder Erdalkalihydroxide, -carbonate, -hydride, - alkoholate und organische Basen, wie Trialkylamine, z.B. Triethyl- oder Tributylamin, quartäre Ammonium- oder Phosphoniumhydroxide und vernetzte Harze mit fixierten, gegebenenfalls substituierten Ammonium- oder Phosphoniumgruppen. Die Xanthinderivate können aber auch unmittelbar in Form ihrer gesondert hergestellten Salze, etwa der Alkali-, Erdalkali- oder gegebenenfalls substituierten Ammonium- oder Phosphoniumsalze, eingesetzt werden. Weiterhin lassen sich die Xanthinverbindungen sowohl in Gegenwart der vorgenannten anorganischen Kondensationsmittel als auch in Form ihrer Alkalioder Erdalkalisalze unter Mithilfe von sogenannten Phasentransferkatalysatoren, beispielsweise tertiären Aminen, quartären Ammonium- oder Phosphoniumsalzen oder auch Kronenethern, bevorzugt in einem zweiphasigen System unter den Bedingungen einer Phasentransferkatalyse, bequem alkylieren. Geeignete, zumeist kommerziell erhältliche Phasentransferkatalysatoren sind unter anderen Tetra(C₁-C₄)alkyl- und Methyltrioctylammonium- und -phosphonium-, Methyl-, Myristyl-, Phenyl- und Benzyl-tri(C₁-C₄)alkyl- und Cetyltrimethylammoniumsowie (C₁-C₁₂)Alkyl- und Benzyl-triphenylphosphoniumsalze, wobei sich in der Regel jene Verbindungen, die das größere und symmetrischer gebaute Kation besitzen, als effektiver erweisen.
Bei den voranstehend beschriebenen Verfahrensweisen wird im allgemeinen bei einer Reaktionstemperatur zwischen 0°C und dem Siedepunkt des jeweils verwendeten Reaktionsmedium gearbeitet, vorzugsweise zwischen 20° und 130°C, gegebenenfalls bei erhöhtem oder vermindertem Druck, aber gewöhnlich bei Atmosphärendruck, wobei die Reaktionszeit von weniger als einer Stunde bis zu mehreren Stunden betragen kann.

Die Eliminierung der Abgangsgruppe R^{a} aus den Verbindungen der Formel VIII unter Bildung der erfindungsgemäßen Xanthine der Formel I erfolgt unter Standardbedingungen, die vor allem im Rahmen der Schutzgruppen-Technik bei Alkaloid- und Peptidsynthesen entwickelt wurden und somit als weitgehend bekannt vorausgesetzt werden können.

Danach wird die gegebenenfalls in den Phenylringen substituierte Benzyl-, Benzhydryl- oder Tritylgruppe vorzugsweise reduktiv abgespalten. Neben der chemischen Reduktion insbesondere der Benzylverbindungen mit Natrium in flüssigem Ammoniak kommt hierfür bevorzugt die Eliminierung der drei vorgenannten Aralkylgruppen durch katalytische Hydrogenolyse mit Hilfe eines Edelmetall-Katalysators in Betracht, wobei sich häufig der Ersatz von molekularem Wasserstoff durch Ammoniumformiat als Wasserstoffdonator bewährt. Als Reaktionsmedium dient hierbei gewöhnlich ein niederer Alkohol, gegebenenfalls unter Zusatz von Ameisensäure oder auch Ammoniak; ein aprotisches Lösungsmittel, wie Dimethylformamid; oder insbesondere Eisessig; aber auch deren Gemische mit Wasser können Verwendung finden. Geeignete Hydrierungskatalysatoren sind vornehmlich Palladium-Schwarz und Palladium auf Aktivkohle oder Bariumsulfat, während andere Edelmetalle, wie Platin, Rhodium und Ruthenium aufgrund konkurrierender Kernhydrierung häufig Anlaß zu Nebenreaktionen geben und deshalb nur bedingt einsetzbar sind. Die Hydrogenolyse wird zweckmäßig bei Temperaturen zwischen 20° und 100°C und unter Atmosphärendruck oder bevorzugt leichtem Überdruck bis zu etwa 10 bar durchgeführt, wobei in der Regel Reaktionszeiten von einigen Minuten bis zu mehreren Stunden benötigt werden.
Wahlweise kann aber die Abspaltung der Schutzgruppe R^{a}, wie etwa des 4-Methoxybenzyl-, Benzhydryl- oder Tritylrestes, auch hydrolytisch unter üblicher Protonenkatalyse erfolgen.

Die Darstellung der erfindungsgemäßen Verbindungen der Formel I in stereoisomerenreiner Form erfolgt bevorzugt durch nachträgliche Trennung der stereoisomeren Formen mit Hilfe an sich bekannter Methoden. Da Diastereomere im Gegensatz zu Enantiomeren unterschiedliche physikalische und chemische Eigenschaften aufweisen, bereitet die Trennung ihrer Gemische, beispielsweise durch fraktionierende Kristallisation oder chromatographische Verfahren, in der Regel keine Schwierigkeiten. Demgegenüber erfordert die physikalische Racematspaltung in die enantiomeren Formen (Antipoden) zusätzliche Vorkehrungen; so gelingt die fraktionierende Kristallisation erst nach Bildung diastereomerer Salze mit einer optisch aktiven Base und die chromatographische Trennung nur bei Verwendung chiraler stationärer Phasen, die zu den Enantiomeren eine unterschiedliche räumliche Affinität zeigen.

Die 1,3,7-trisubstituierten Xanthine der Formel VIII stellen wertvolle Zwischenprodukte zur Herstellung der erfindungsgemäßen Verbindungen der Formel I dar, lassen darüber hinaus, insbesondere wenn R^{a} Benzyl bedeutet, bereits dieselbe pharmakologische Wirkungsrichtung wie die Endprodukte der Formel I erkennen und gehören daher ebenfalls zum Anspruchsbereich der vorliegenden Erfindung, wenngleich sie aufgrund ihrer geringeren Wasserlöslichkeit schwieriger parenteral zu verabreichen sind.

Die erfindungsgemäßen neuen Verbindungen der Formel I eignen sich aufgrund ihrer wertvollen pharmakologischen Eigenschaften in hervorragender Weise für die Verwendung als Wirkstoffe in Arzneimitteln, insbesondere in solchen, die eine effektive kurative und prophylaktische Behandlung von Schockerkrankungen gestatten und somit eine wesentliche Bereicherung des Arzneimittelschatzes darstellen. Sie können entweder für sich allein, beispielsweise in Form von Mikrokapseln, in Mischungen untereinander oder in Kombination mit geeigneten Trägerstoffen verabreicht werden.

Die Erfindung betrifft daher auch Arzneimittel, die mindestens eine Verbindung der Formel I als Wirkstoff enthalten, wobei die als Wirkstoffe für Arzneimittel mit anderer Indikationsstellung vorbeschriebenen 3-n-Propylxanthine mit 2-Methoxyethyl, 2-Ethoxyethyl oder 3-Methoxypropyl in 1-Position ausgenommen sind.

Ein weiterer Aspekt der vorliegenden Erfindung ist die Verwendung der Verbindung der Formel I zur Herstellung von pharmazeutischen Zubereitungen für die parenterale und orale, gegebenenfalls aber auch rektale, transdermale oder inhalative Verabreichung bei Schockerkrankungen. Geeignete feste oder flüssige galenische Zubereitungsformen sind beispielsweise Granulate, Pulver, Tabletten, Dragees, (Mikro)Kapseln, Sirupe, Emulsionen, Suspensionen, Gele, Präparate mit protrahierter Wirkstoff-Freigabe, Zäpfchen, wirkstoffabgebende Pflaster, Aerosole, Tropfen und vor allem injizierbare Lösungen in Form von Ampullen oder Injektionsflaschen für die Dauerinfusion, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, physiologische Kochsalzlösung, Alkohole, Glycerin und andere mehrwertige Alkohole (Polyole) genannt.

Vorzugsweise werden die pharmazeutischen Präparate in Dosierungseinheiten hergestellt und verabreicht, wobei jede Einheit als aktiven Bestandteil eine bestimmte Dosis einer Verbindung gemäß Formel I enthält. Bei festen Dosierungseinheiten, wie Tabletten, Kapseln und Suppositorien, kann diese Dosis bis zu 1000 mg, bevorzugt jedoch 100 bis 600 mg, und bei Injektionslösungen in Ampullenform bis zu 300 mg, vorzugsweise aber 20 bis 200 mg, betragen.

Für die Behandlung eines erwachsenen Patienten sind - je nach Wirksamkeit der Verbindungen gemäß Formel I am Menschen und Schweregrad der lebensbedrohlichen Erkrankung - Tagesdosen von 100 bis 5000 mg Wirkstoff, vorzugsweise 300 bis 3000 mg, bei oraler Verabreichung und von 30 bis 3000 mg, bevorzugt 50 bis 2000 mg, bei intravenöser Applikation indiziert. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Zeitintervallen erfolgen.

Bei intravenöser Dauerinfusion beträgt die Tagesdosis 100 bis 5000 mg, vorzugsweise 500 bis 2000 mg, entsprechend einer Infusionsgeschwindigkeit von 0,1 bis 3 mg pro kg Körpergewicht und Stunde (h), bevorzugt von 0,3 bis 1 mg/kg/h.

Bei allen Applikationsformen können jedoch unter Umständen auch höhere oder niedrigere Tagesdosen angebracht sein.

Schließlich können die Verbindungen der Formel I - falls klinisch indiziert - auch zusammen mit anderen geeigneten Wirkstoffen, insbesondere mit solchen, die ebenfalls regulierend in die Signalkaskade des Schockgeschehens eingreifen; beispielsweise mit Antikörpern gegen Entero- und Endotoxine (LPS), dem monozytären LPS-Rezeptor CD14 oder dem LPS-bindenden Protein LBP; mit Modulatoren des Zytokin-Netzwerkes wie Anti-TNF-Antikörpern, löslichen TNF-Rezeptoren und anderen TNF-bindenden Proteinen, Inhibitoren der Interleukin-1(IL-1)- und/oder TNF-Produktion und/oder -Freisetzung sowie TNF- und IL-1-Rezeptor-Antagonisten; mit Hemmstoffen des Arachidonsäure-Stoffwechsels sowie der Koagulations- und Komplementkaskade wie Phospholipase A₂-, Cyclooxygenase- und Lipoxygenase-Inhibitoren (z.B. Steroiden und nichtsteroidalen Antiphlogistika wie Ibuprofen), PAF(plättchenaktivierender Faktor)-, Leukotrien-, Thromboxan-, Thrombin-, Fibrin-, Bradykinin- und Serotonin-Antagonisten sowie Anti-C5a- oder -C3a-Antikörpern; mit Antikoagulantien und Thrombozyten-Aggregationshemmern wie Antithrombin III, dem Gewebe-Plasminogen-Aktivator tPA-1, Heparin sowie Prostazyklin und dessen stabileren synthetischen Derivaten; mit Hemmern der Freisetzung und/oder der biologischen Wirkung von lytischen Enzymen; mit Sauerstoffradikalfängern wie Superoxiddismutase, Katalase, alpha-Tocopherol oder N-Acetylcystein; mit Schwermetall-Chelatoren wie Deferoxamin; mit Inhibitoren der interzellulären Adhäsion wie Fibronektin oder Antikörpern gegen die Adhäsionsmoleküle ELAM-1, ICAM-1, VCAM-1 und CD11/CD18; oder aber auch mit Antibiotika verabreicht oder bei der Herstellung der vorgenannten galenischen Zubereitungsformen gemeinsam formuliert werden.

Nachfolgend wird anhand repräsentativer Herstellungsbeispiele der Aufbau der nach Strukturgesichtspunkten in Tabelle 1 zusammengefaßten Verbindungen gemäß Formel I näher erläutert. In Tabelle 2 sind die wertvollen Zwischenverbindungen der Formel VIII in gleicher Anordnung zusammengestellt. Für alle präparativ hergestellten Zwischen- und Endprodukte wurde die Struktur sowohl ¹H-NMR-spektroskopisch als auch durch Elementaranalyse bzw. Massenspektrum gesichert.

### Herstellungsbeispiele

### Beispiel 1: 1-Methoxymethyl-3-methylxanthin (Verbindung 1)

### a) 7-Benzyl-3-methylxanthin

Zu einer Suspension von 83 g (0,5 mol) 3-Methylxanthin in 500 ml Methanol gab man 20 g (0,5 mol) in 200 ml Wasser gelöstes Natriumhydroxid und rührte eine Stunde bei 70°C, versetzte dann bei derselben Temperatur tropfenweise mit 85,5 g (0,5 mol) Benzylbromid und hielt das Reaktionsgemisch für 5 Stunden zwischen 70° und 80°C. Anschließend wurde abgekühlt, kalt abgenutscht, das Produkt auf der Nutsche mit Wasser gewaschen, in 1000 ml 1 N Natronlauge heiß gelöst, filtriert und mit 4 N Salzsäure unter Rühren langsam auf pH 9,5 gebracht. Man filtrierte das Kristallisat von der noch warmen Lösung ab, wusch mit Wasser chloridfrei und trocknete im Vakuumschrank. Ausbeute: 81,7 g (63,8% der Theorie); Schmelzpunkt: 263°C C₁₃H₁₂N₄O₂ (MG = 256,2 g/mol)

### b) 7-Benzyl-1-methoxymethyl-3-methylxanthin

2,3 g (0,1 g-atom) Natrium wurden in 200 ml wasserfreiem Methanol gelöst, mit 25,6 g (0,1 mol) Xanthin aus Stufe a) versetzt, bis zur klaren Lösung unter Rückfluß erhitzt, dann abgekühlt, unter vermindertem Druck eingedampft und getrocknet. Das so gewonnene Natriumsalz des 7-Benzyl-3-methylxanthins suspendierte man in 300 ml wasserfreiem Acetonitril, fügte unter Rühren bei 50°C eine Lösung von 8,8 g (0,11 mol) Methoxymethylchlorid in 40 ml Acetonitril tropfenweise hinzu und rührte 8 Stunden bei 50°C nach. Anschließend wurde abgekühlt, unter vermindertem Druck eingedampft, der Rückstand in Chloroform aufgenommen, unumgesetztes 7-Benzyl-3-methylxanthin mit 1 N Natronlauge ausgeschüttelt, die Chloroformphase mit Wasser neutralgewaschen, getrocknet und unter vermindertem Druck eingedampft, wobei 22 g (73,3% der Theorie) öliges Produkt anfielen, das sich allmählich verfestigte und aus Essigsäureethylester unter Zusatz von Petrolether in der Siedehitze umkristallisieren ließ.
C₁₅H₁₆N₄O₃ (MG = 300,3 g/mol); Schmelzpunkt: 114°C
Die Einführung der Methoxymethylgruppe in die 1-Stellung des 7-Benzyl-3-methylxanthins gelingt auch mit Methoxymethyl-4-toluolsulfonat als Alkylierungsmittel, das in einer Eintopfreaktion aus 4-Toluolsulfonsäurechlorid und Natriumacetat oder 4-Toluolsulfonsäure und Acetanhydrid mit Formaldehyddimethylacetal in Dimethylformamid erzeugt (WO 87/00523) und in situ mit 7-Benzyl-3-methylxanthin umgesetzt wird.

### c) 1-Methoxymethyl-3-methylxanthin (Verbindung 1)

10,5 g (0,035 mol) des 1,3,7-trisubstituierten Xanthins aus Stufe b) wurden in 200 ml Eisessig über 1,5 g Palladium (10%) auf Aktivkohle bei 60°C und 3,5 bar in 48 Stunden hydriert. Nach Abkühlen überlagerte man mit Stickstoff, filtrierte den Katalysator ab, engte unter vermindertem Druck ein und reinigte den festen Rückstand durch Filtration über eine Kieselgel-Säule im Fließmittel Chloroform/Methanol (10/1).
Ausbeute: 5,5 g (74,8% der Theorie); Schmelzpunkt: 218°C
C₈H₁₀N₄O₃ (MG = 210,2 g/mol)

| | | | | |
|---|---|---|---|---|
| Analyse | Berechnet | C 45,71% | H 4,80% | N 26,66% |
| | Gefunden | C 45,98% | H 4,86% | N 26,98% |

### Beispiel 2: 3-Cyclopropyl-1-(2-methoxyethyl)-xanthin (Verbindung 10)

### a) 7-Benzyl-3-cyclopropylxanthin

Zu einer Suspension von 50 g (0,26 mol) 3-Cyclopropylxanthin in 300 ml Methanol gab man 10,4 g (0,26 mol) in 110 ml Wasser gelöstes Natriumhydroxid und rührte eine Stunde bei 70°C, versetzte dann bei derselben Temperatur tropfenweise mit 44,5 g (0,26 mol) Benzylbromid und hielt das Reaktionsgemisch für 4 Stunden zwischen 70° und 80°C. Dann gab man 1,04 g (0,026 mol) Natriumhydroxid und 4,45 g (0,026 mol) Benzylbromid zu. Nach einer weiteren Stunde wurde abgekühlt, kalt abgenutscht und das Produkt auf der Nutsche mit Wasser gewaschen. Das so erhaltene Rohprodukt konnte ohne weitere Reinigung eingesetzt werden.
Ausbeute: 48 g (65,4% der Theorie); Schmelzpunkt: 204°C
C₁₅H₁₄N₄O₂ (MG = 282,3 g/mol); Massenspektrum: 283 (60%, M+H); 240 (21%); 91 (100%)

### b) 7-Benzyl-3-cyclopropyl-1-(2-methoxyethyl)-xanthin

Zu einer 60°C heißen Lösung von 3 g (11,0 mmol) 7-Benzyl-3-cyclopropylxanthin aus Stufe a) in Dimethylformamid gab man 2,2 g (15,9 mmol) Kaliumcarbonat und rührte 1 Stunde bei 60°C. Dann gab man tropfenweise 1,51 g (15,9 mmol) 2-Methoxyethylchlorid hinzu und rührte 6 Stunden bei 80°C. Anschließend ließ man auf Raumtemperatur abkühlen und engte unter vermindertem Druck ein. Der ölige Rückstand wurde in Dichlormethan aufgenommen und mit 1 N Natronlauge extrahiert, mit Wasser neutral gewaschen, über Magnesiumsulfat getrocknet, unter vermindertem Druck eingeengt und ohne weitere Reinigung in Stufe c) eingesetzt.
Ausbeute: 2,7 g (71,7% der Theorie); Schmelzpunkt: 117°C
C₁₈H₂₀N₄O₃ (MG = 340,4 g/mol); Massenspektrum: 340 (36%, M); 282 (43%); 148 (100%); 91 (86%)

### c) 3-Cyclopropyl-1-(2-methoxyethyl)-xanthin (Verbindung 10)

2,2 g (6,45 mmol) des 1,3,7-trisubstituierten Xanthins aus Stufe b) wurden in 250 ml Ethanol über 1,05 g Palladium (10%) auf Aktivkohle in 12 Stunden hydriert. Man überlagerte mit Stickstoff, filtrierte den Katalysator ab, engte unter vermindertem Druck ein und reinigte flash-chromatographisch über eine Kieselgel-Säule, Toluol/Ethanol (10/1).
Ausbeute: 0,77 g (47,7% der Theorie); Schmelzpunkt: 203°C
C₁₁H₁₄N₄O₃ (MG = 250,3 g/mol); Massenspektrum: 250 (55%, M); 192 (100%); 149 (56%); 148 (58%); 121 (82%); 120 (56%)

### Beispiel 3: 3-Butyl-1-(3-methoxypropyl)-xanthin (Verbindung 14)

### a) 7-Benzyl-3-butylxanthin

Zu einer Suspension von 52 g (0,25 mol) 3-Butylxanthin in 300 ml Methanol gab man 10 g (0,25 mol) in 100 ml Wasser gelöstes Natriumhydroxid und rührte eine Stunde bei 70°C, versetzte dann bei derselben Temperatur tropfenweise mit 42,8 g (0,25 mol) Benzylbromid und hielt das Reaktionsgemisch für 5 Stunden zwischen 70° und 80°C. Dann gab man 1,0 g (0,025 mol) Natrium-hydroxid und 4,28 g (0,025 mol) Benzylbromid zu. Nach weiteren 2 Stunden wurde abgekühlt, mit 1500 ml Wasser verdünnt, kalt abgenutscht, das Produkt auf der Nutsche mit Wasser gewaschen, in 1000 ml 1 N Natronlauge gelöst, filtriert und mit konzentrierter Salzsäure unter Rühren langsam auf pH 3 gebracht. Man filtrierte das Kristallisat von der Lösung ab, wusch mit Wasser chloridfrei und trocknete unter verminderten Druck. Ausbeute: 54,1 g (72,5% der Theorie); Schmelzpunkt: 187°C
C₁₆H₁₈N₄O₂ (MG = 298,3 g/mol); Massenspektrum: 298 (13%, M); 91 (100%)

### b) 7-Benzyl-3-butyl-1-(3-methoxypropyl)-xanthin

Zu einer 60°C heißen Lösung von 3 g (10,0 mmol) 7-Benzyl-3-butylxanthin aus Stufe a) in 90 ml Dimethylformamid gab man 2,1 g (15,2 mmol) Kaliumcarbonat und rührte 1 Stunde bei 60°C. Dann gab man tropfenweise 1,3 g (12,0 mol) 3-Methoxypropylchlorid zu und rührte 3 Stunden bei 100°C. Anschließend ließ man auf Raumtemperatur abkühlen, versetzte mit Wasser und extrahierte mit Dichlormethan. Die organische Phase wurde mit Wasser und 1 N Natronlauge gewaschen, mit Natriumsulfat getrocknet und unter vermindertem Druck eingeengt. Der ölige Rückstand wurde flash-chromatographisch über eine Kieselgel-Säule gereinigt, Toluol/Ethanol (39/1).
Ausbeute: 3,2 g (86,5% der Theorie); gelbes Öl
C₂₀H₂₆N₄O₃ (MG = 370,5 g/mol); Massenspektrum: 371,3 (100%, M+H); 339,3 (16%); 298,3 (15%); ¹H-NMR (DMSO-d₆, 200 MHz): δ = 0,90 (t, 3 H, CH₂CH₃); 1,30 (sixt., 2 H, CH₂CH₂CH₃); 1,63 u. 1,75 (2 quint., 4 H, CH₂CH₂CH₂); 3,32 (s, 3 H, OCH₃); 3,34 (t, 2 H, OCH₂); 5,48 (s, 2 H, benz. H); 7,25-7,40 (m, 5 H, aromat. H); 8,26 (s, 1 H, N=CH)

### c) 3-Butyl-1-(3-methoxypropyl)-xanthin (Verbindung 14)

0,5 g (1,35 mmol) des 1,3,7-trisubstituierten Xanthins aus Stufe b) wurden in 50 ml Ethanol über 0,1 g Palladium (10%) auf Aktivkohle in 5 Stunden hydriert. Man überlagerte mit Stickstoff, filtrierte den Katalysator ab und engte unter vermindertem Druck ein. Der Rückstand konnte aus Methanol/Methyl-tert.butylether umkristallisiert werden.
Ausbeute: 0,19 g (52,2% der Theorie); Schmelzpunkt: 157°C
C₁₃H₂₀N₄O₃ (MG = 280,3 g/mol); Massenspektrum: 281,3 (M + H, 100%); 249,2 (M-OMe, 70%)

### Beispiel 4: 1-Ethoxymethyl-3-propylxanthin (Verbindung 18)

### a) 7-Benzyl-3-propylxanthin

Zu einer Suspension von 20 g (0,103 mol) 3-Propylxanthin in 112 ml Methanol gab man 4,12 g (0,103 mol) in 41 ml Wasser gelöstes Natriumhydroxid und rührte eine Stunde bei 70°C, versetzte dann bei derselben Temperatur tropfenweise mit 12,23 ml (0,103 mol) Benzylbromid und hielt das Reaktionsgemisch für 4 Stunden zwischen 70° und 80°C. Es wurde abgekühlt, kalt abgenutscht, das Produkt auf der Nutsche mit Wasser gewaschen und unter verminderten Druck getrocknet.
Ausbeute: 20,3 g (69,4% der Theorie); Schmelzpunkt: 186°C
C₁₅H₁₆N₄O₂ (MG = 284,3 g/mol); Massenspektrum: 284 (18%, M); 242 (11%); 212 (13%); 91 (100%)

### b) 7-Benzyl-1-ethoxymethyl-3-propylxanthin

Zu einer 60°C heißen Lösung von 2,2 g (7,7 mmol) 7-Benzyl-3-propylxanthin aus Stufe a) in 60 ml Dimethylformamid gab man 1,71 g (12,0 mmol) Kaliumcarbonat und rührte 1 Stunde bei 60°C. Dann gab man tropfenweise 0,93 ml (10,0 mmol) Ethoxymethylchlorid zu und rührte 4,5 Stunden bei 80°C. Man gab weitere 0,5 ml (5,3 mmol) Ethoxymethylchlorid zu und rührte erneut 6 Stunden. Anschließend wurden 12 ml Wasser und 5 ml Methanol zugegeben, über Nacht stehen gelassen, erneut 60 ml Wasser zugegeben und dreimal mit Methyl-tert.-butylether extrahiert. Die vereinigten organischen Phasen wurden zweimal mit Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (19,8/0,2). Ausbeute: 2,28 g (87% der Theorie); Schmelzpunkt: 110°C
C₁₈H₂₂N₄O₃ (MG = 342,4 g/mol); Massenspektrum: 342 (7%, M); 296 (13%); 285 (33%); 91 (100%)

### c) 1-Ethoxymethyl-3-propylxanthin (Verbindung 18)

1,79 g (5,2 mmol) des 1,3,7-trisubstituierten Xanthins aus Stufe b) wurden in 200 ml Ethanol über 179 mg Palladium (10%) auf Aktivkohle in 6,5 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/Methanol, (19,8/0,2). Ausbeute: 1,12 g (85% der Theorie); Schmelzpunkt: 134°C
C₁₁H₁₆N₄O₃ (MG = 252,3 g/mol); Massenspektrum: 252 (29%, M); 208 (40%); 195 (100%); 166 (65%); 136 (50%)

### Beispiel 5: 3-Ethyl-1-propoxymethylxanthin (Verbindung 27)

### a) 7-Benzyl-3-ethylxanthin

180 g (1 mol) 3-Ethylxanthin wurden in 1000 ml Dimethylformamid vorgelegt, unter Rühren auf 80°C erwärmt und nach dem Eintragen von 88 g (0,64 mol) Kaliumcarbonat tropfenweise mit 133 g (1,05 mol) Benzylchlorid innerhalb 1 Stunde versetzt. Danach wurde für 2 Stunden bei 100°C gerührt, mit 1000 ml Wasser versetzt, das ausgefallene Produkt abgenutscht, mit Wasser salzfrei gewaschen und im Vakuumschrank bei 100°C getrocknet. Falls erforderlich, kann eine weitere Reinigung durch Umfällen aus 1 N Natronlauge mit 4 N Salzsäure analog Beispiel 1a) vorgenommen werden.
Ausbeute: 262 g (97% der Theorie); Schmelzpunkt: 218°C
C₁₄H₁₄N₄O₂ (MG = 270,3 g/mol)

| | | | | |
|---|---|---|---|---|
| Analyse | Berechnet | C 62,21% | H 5,22% | N 20,73% |
| | Gefunden | C 62,07% | H 5,36% | N 20,84% |

### b) 7-Benzyl-3-ethyl-1-propoxymethylxanthin

Analog Beispiel 1 b) wurden 27 g (0,1 mol) 7-Benzyl-3-ethylxanthin in das Natriumsalz umgewandelt, anschließend in Acetonitril mit 13 g (0,12 mol) Propoxymethylchlorid (hergestellt in 67%-iger Ausbeute aus 1,3,5-Trioxan, 1-Propanol und Chlorwasserstoff-Gas) umgesetzt und aufgearbeitet, wobei 30 g (87,6% der Theorie) analysenreines Produkt anfielen, das sich gegebenenfalls aus Essigsäureethylester umkristallisieren ließ.
C₁₈H₂₂N₄O₃ (MG = 342,4 g/mol); Schmelzpunkt: 92°C

| | | | | |
|---|---|---|---|---|
| Analyse | Berechnet | C 63,14% | H 6,48% | N 16,36% |
| | Gefunden | C 62,95% | H 6,55% | N 16,21% |

### c) 3-Ethyl-1-propoxymethylxanthin (Verbindung 27)

17,1 g (0,05 mol) des Produktes aus Stufe b) und 5 g (0,08 mol) Ammoniumformiat wurden in 150 ml Ethanol über 6 g Palladium (10%) auf Aktivkohle bei 35°C für mehrere Tage gerührt, wobei sich ein sukzessiver Zusatz von weiterem Ammoniumformiat bis zu einer Gesamtmenge von 22 g (0,35 mol) bewährte. Es wurde filtriert, das Filtrat eingeengt, der Rückstand in Natriumcarbonat-Lösung aufgenommen, mit Chloroform gewaschen, die wäßrige Phase mit 2 N Salzsäure auf pH 4 gebracht, das Produkt mit Chloroform ausgeschüttelt und nach Trocknen und Eindampfen aus Essigsäureethylester umkristallisiert.
Ausbeute: 8,6 g (68,2% der Theorie); Schmelzpunkt: 159°C
C₁₁H₁₆N₄O₃ (MG = 252,3 g/mol)

| | | | | |
|---|---|---|---|---|
| Analyse | Berechnet | C 52,37% | H 6,39% | N 22,21% |
| | Gefunden | C 52,85% | H 6,88% | N 22,50% |

Die hydrogenolytische Debenzylierung analog Beispiel 1 c) lieferte dieselbe Verbindung in 58,9%-iger Ausbeute.

### Beispiel 6: 3-Isobutyl-1-propoxymethylxanthin (Verbindung 31)

### a) 7-Benzylguanin-hydrochlorid

Zu einer Suspension von 40 g (0,147 mol) Guanosin in 200 ml Dimethylsulfoxid gab man tropfenweise 40 ml (0,34 mol) Benzylbromid und rührte 4 Stunden bei Raumtemperatur. Es wurde mit 100 ml konzentrierter Salzsäure versetzt und 30 Minuten bei Raumtemperatur gerührt. Anschließend goß man in 1200 ml Methanol, saugte den Niederschlag ab und wusch mit Methanol.
Ausbeute: 35,9 g (92% der Theorie); Schmelzpunkt: >325°C
C₁₂H₁₂ClN₅O (MG = 277,7 g/mol); Base: C₁₂H₁₁N₅O (MG = 241,6 g/mol) Massenspektrum: 242,2 (100%, M+H)

### b) 7-Benzylxanthin

35,9 g (0,13 mol) 7-Benzylguanin-hydrochlorid aus Stufe a) wurden in einer Mischung aus 90 ml Wasser und 807 ml Eisessig gelöst und auf 100°C erwärmt. Nach Abkühlen auf 50°C gab man eine Lösung von 35,88 g (0,52 mol) Natriumnitrit in 90 ml Wasser auf einmal hinzu. Nach 16 Stunden bei Raumtemperatur wurde der entstandene Niederschlag abgesaugt, auf der Nutsche mit Wasser gewaschen und getrocknet.
Ausbeute: 26,0 g (83% der Theorie); Schmelzpunkt: >266°C
C₁₂H₁₀N₄O₂ (MG = 242,5 g/mol); Massenspektrum: 243,1 (95%, M+H); 91 (100%)

### c) 7-Benzyl-3-isobutylxanthin

1,5 g (6,2 mmol) 7-Benzylxanthin aus Stufe b) wurden in 50 ml Dimethylformamid bei 50°C gelöst und portionsweise mit 0,149 g (6,2 mmol) Natriumhydrid versetzt und eine Stunde bei 50°C gerührt. Man gab tropfenweise 0,67 ml (6,2 mmol) Isobutylbromid zu und erwärmte auf 80°C. Nach 5 Stunden gab man weitere 0,2 ml (1,86 mmol) Isobutylbromid zu und rührte weitere 5 Stunden. Anschließend gab man 12 ml Wasser und 5 ml Methanol hinzu, rührte 2 Stunden bei Raumtemperatur, gab weitere 60 ml Wasser hinzu und extrahierte dreimal mit Methyl-tert.-butylether. Die organischen Phasen wurden mit Wasser gewaschen, mit Magnesiumsulfat getrocknet, unter vermindertem Druck eingeengt und flash-chromatographisch über eine Kieselgel-Säule gereinigt, Dichlormethan/Methanol (99/1).
Ausbeute: 1,16 g (63% der Theorie); C₁₆H₁₈N₄O₂ (MG = 298,3 g/mol)
¹H-NMR (DMSO-d₆, 200 MHz): δ = 0,85 (d, 6 H, CH(CH₃)₂); 2,16 (m, 1 H, CH₂CH(CH₃)₂); 3,73 (d, 2 H, CH₂CH); 5,45 (s, 2 H, benzyl. H); 7,23-7,40 (m, 5 H, aromat. H), 8,20 (s, 1 H, N=CH); 11,13 (s br., 1 H, NH)

### d) 7-Benzyl-3-isobutyl-1-propoxymethylxanthin

Zu einer Suspension von 1,16 g (3,9 mmol) 7-Benzyl-3-isobutylxanthin aus Stufe c) in 60 ml Dimethylformamid gab man bei 60°C 0,86 g (6,2 mmol) Kaliumcarbonat und rührte eine Stunde bei dieser Temperatur. Dann gab man tropfenweise 0,56 ml (5,1 mmol) Propoxymethylchlorid hinzu und rührte 5,5 Stunden bei 80°C. Anschließend wurden 12 ml Wasser und 5 ml Methanol zugegeben, über Nacht stehen gelassen, erneut 60 ml Wasser zugegeben und viermal mit je 150 ml Methyl-tert.-butylether extrahiert. Die vereinigten organischen Phasen wurden mit 200 ml Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan.
Ausbeute: 1,2 g (83% der Theorie); Schmelzpunkt: 72°C
C₂₀H₂₆N₄O₃ (MG = 370,5 g/mol); Massenspektrum: 370 (40%, M); 310 (55%); 299 (100%); 256 (55%); 91 (85%)

### e) 3-lsobutyl-1-propoxymethylxanthin (Verbindung 31)

859 mg (2,32 mmol) des 1,3,7-trisubstituierten Xanthins aus Stufe d) wurden in 22 ml Ethanol über 86 mg Palladium (10%) auf Aktivkohle in 6 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/Methanol, (19/1).
Ausbeute: 588 mg (90% der Theorie); Schmelzpunkt: 141°C
C₁₃H₂₀N₄O₃ (MG = 280,3 g/mol); Massenspektrum: 280 (25%, M); 222 (37%); 209 (100%); 166 (85%); 136 (55%)

### Beispiel 7: 3-Phenyl-1-propoxymethylxanthin (Verbindung 32)

### a) 7-Benzyl-3-phenylxanthin

Zu einer Suspension von 3,0 g (13,2 mmol) 3-Phenylxanthin in 18 ml Methanol gab man eine Lösung von 0,53 g (13,2 mmol) Natriumhydroxid in 5,3 ml Wasser und rührte eine Stunde bei 70°C. Dann versetzte man tropfenweise mit 1,56 ml (13,2 mmol) Benzylbromid, rührte 7 Stunden bei 70°C, saugte nach dem Erkalten den Niederschlag ab, wusch mit Wasser, löste den Niederschlag in 50 ml 1 N Natronlauge, filtrierte vom Unlöslichen ab und stellte mit 4 N Salzsäure auf pH 8-9. Der entstandene Niederschlag wurde abgesaugt, mit Wasser gewaschen und flash-chromatographisch über eine Kieselgel-Säule gereinigt, Dichlormethan/Methanol (79/1).
Ausbeute: 1,13 g (27% der Theorie); Schmelzpunkt: 250°C
C₁₈H₁₄N₄O₂ (MG = 318,6 g/mol); Massenspektrum: 319 (100%, M+H); 91 (19%)

### b) 7-Benzyl-3-phenyl-1-propoxymethylxanthin

Zu einer Suspension von 0,65 g (2,04 mmol) 7-Benzyl-3-phenylxanthin aus Stufe a) in 20 ml Dimethylformamid gab man bei 60°C 0,45 g (3,26 mmol) Kaliumcarbonat und rührte eine Stunde bei dieser Temperatur. Dann gab man tropfenweise 0,29 ml (2,65 mmol) Propoxymethylchlorid hinzu und rührte 1,5 Stunden bei 80°C. Anschließend wurden 20 ml Wasser zugegeben, dreimal mit je 24 ml Methyl-tert.-butylether extrahiert, die vereinigten organischen Phasen zweimal mit je 12 ml Wasser gewaschen, mit Magnesiumsulfat getrocknet und unter vermindertem Druck eingeengt. Das Rohprodukt wurde flash-chromato-graphisch über eine Kieselgel-Säule gereinigt, Heptan/Ethylacetat (5/7).
Ausbeute: 0,69 g (87% der Theorie); Schmelzpunkt: 103°C
C₂₂H₂₂N₄O₃ (MG = 390,4 g/mol); Massenspektrum: 391,2 (100%, M+H); 331,2 (12%); 241,1 (25%)

### c) 3-Phenyl-1-propoxymethylxanthin (Verbindung 32)

535 mg (1,37 mmol) des 1,3,7-trisubstituierten Xanthins aus Stufe b) wurden in 20 ml Ethanol über 50 mg Palladium (10%) auf Aktivkohle hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/Methanol (19/0,3).
Ausbeute: 232 mg (56% der Theorie); Schmelzpunkt: 220°C
C₁₅H₁₆N₄O₃ (MG = 300,3 g/mol); Massenspektrum: 300 (23%, M); 242 (68%); 229 (55%); 185 (100%)

### Beispiel 8: 3-Cyclopropylmethyl-1-propoxymethylxanthin (Verbindung 34)

### a) 7-Benzyl-3-cyclopropylmethylxanthin

Eine Lösung von 7 g (29,0 mmol) 7-Benzylxanthin aus Beispiel 6 b) in 200 ml Dimethylformamid wurde auf 50°C erwärmt und portionsweise mit 0,69 g (29,0 mmol) Natriumhydrid versetzt und eine Stunde bei 50°C gerührt. Zu dieser Suspension gab man 2,76 ml (29,0 mmol) Cyclopropylmethylbromid und erhöhte die Temperatur auf 80°C. Nach 7 Stunden bei 80°C gab man erneut 1 ml (11,0 mmol) Cyclopropylmethylbromid hinzu. Nach weiteren 6 Stunden gab man 24 ml Wasser und 10 ml Methanol zu, ließ über Nacht stehen, versetzte erneut mit 120 ml Wasser und extrahierte dreimal mit je 300 ml Methyl-tert.-butylether. Die organischen Phasen wurden mit Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (99/1).
Ausbeute: 4,8 g (56% der Theorie); Schmelzpunkt: 185°C
C₁₆H₁₆N₄O₂ (MG = 296,4 g/mol); Massenspektrum: 297,3 (100%, M+H)

### b) 7-Benzyl-3-cyclopropylmethyl-1-propoxymethylxanthin

In eine Lösung von 1,5 g (5,06 mmol) 7-Benzyl-3-cyclopropylmethylxanthin aus Stufe a) in 60 ml Dimethylformamid gab man bei 60°C 1,12 g (8,1 mmol) Kaliumcarbonat und rührte eine Stunde bei dieser Temperatur. Dann gab man tropfenweise 722 µl (6,58 mmol) Propoxymethylchlorid hinzu und rührte 4 Stunden bei 80°C. Man gab 12 ml Wasser und 5 ml Methanol zu und rührte 2 Stunden bei 50°C. Anschließend wurden erneut 60 ml Wasser zugegeben, dreimal mit Methyl-tert.-butylether extrahiert, die vereinigten organischen Phasen zweimal mit Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (19,8/0,2).
Ausbeute: 1,32 g (71% der Theorie); Schmelzpunkt: 88°C;
C₂₀H₂₄N₄O₃ (MG = 368,4 g/mol); Massenspektrum: 368 (9%, M); 310 (11%); 297 (13%); 91 (100%)

### c) 3-Cyclopropylmethyl-1-propoxymethylxanthin (Verbindung 34)

938 mg (2,55 mmol) des 1,3,7-trisubstituierten Xanthins aus Stufe b) wurden in 60 ml Ethanol über 130 mg Palladium (10%) auf Aktivkohle in 15 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/ Methanol (39/1).
Ausbeute: 671 mg (95% der Theorie); Schmelzpunkt: 132°C
C₁₃H₁₈N₄O₃ (MG = 278,3 g/mol); Massenspektrum: 278 (26%, M); 220 (80%); 207 (64%); 136 (87%); 122 (67%); 55 (100%)

### Beispiel 9: 1-(2-Propoxyethyl)-3-propylxanthin (Verbindung 37)

### a) 7-Benzyl-1-(2-propoxyethyl)-3-propylxanthin

Zu einer Suspension von 2,2 g (7,8 mmol) 7-Benzyl-3-propylxanthin (hergestellt gemäß Beispiel 4 a) in 70 ml Dimethylformamid gab man bei 60°C 1,7 g (12,48 mmol) Kaliumcarbonat und rührte eine Stunde bei dieser Temperatur. Dann gab man tropfenweise 1,3 ml (10,14 mmol) 2-Propoxyethylchlorid hinzu und rührte 10 Stunden bei 80°C. Anschließend wurden 1,2 ml Methanol und 14 ml Wasser zugegeben, über Nacht stehen gelassen, mit weiteren 70 ml Wasser versetzt und dreimal mit je 84 ml Methyl-tert.-butylether extrahiert. Die vereinigten organischen Phasen wurden zweimal mit je 42 ml Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (19/0,1).
Ausbeute: 2,3 g (80% der Theorie); Schmelzpunkt: 55°C
C₁₉H₂₄N₄O₃ (MG = 356,4 g/mol); Massenspektrum: 356 (10%, M); 297 (15%); 285 (38%); 91 (100%)

### b) 1-(2-Propoxyethyl)-3-propylxanthin (Verbindung 37)

1,75 g (4,7 mmol) des 1,3,7-trisubstituierten Xanthins aus Stufe a) wurden in 75 ml Ethanol über 0,2 g Palladium (10%) auf Aktivkohle in 6 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/ Methanol (38/1).
Ausbeute: 0,93 g (70% der Theorie); Schmelzpunkt: 137°C; C₁₃H₂₀N₄O₃
(MG = 280,6 g/mol); Massenspektrum: 281,3 (45%, M+H); 221,2 (100%)

### Beispiel 10: 1-Butoxymethyl-3-isopropylxanthin (Verbindung 42)

### a) 7-Benzyl-3-isopropylxanthin

Eine Lösung von 3,5 g (1,45 mmol) 7-Benzylxanthin aus Beispiel 6 b) in 60 ml Dimethylformamid wurde auf 50°C erwärmt und portionsweise mit 0,35 g (1,45 mmol) Natriumhydrid versetzt, mit 20 ml Dimethylformamid verdünnt und eine Stunde bei 50°C gerührt. Zu dieser Suspension gab man 1,36 ml (1,45 mmol) 2-Brompropan und erhöhte die Temperatur auf 80°C. Im Verlauf der Reaktion gab man noch insgesamt 4,91 ml (52,3 mmol) 2-Brompropan hinzu. Nach insgesamt 16 Stunden bei 80°C gab man 10 ml Wasser und 2 ml Methanol zu, ließ 10 Minuten rühren, versetzte mit weiteren 70 ml Wasser und extrahierte dreimal mit je 70 ml Methyl-tert.-butylether. Die organischen Phasen wurden mit Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (19/0,4).
Ausbeute: 1,17 g (29% der Theorie); Schmelzpunkt: 219°C
C₁₅H₁₈N₄O₂ (MG = 286,6 g/mol); Massenspektrum: 285,2 (100%, M+H)

### b) 7-Benzyl-1-butoxymethyl-3-isopropylxanthin

Zu einer Suspension von 0,75 g (2,64 mmol) 7-Benzyl-3-isopropylxanthin aus Stufe a) in 20 ml Dimethylformamid gab man bei 60°C 0,583 g (4,22 mmol) Kaliumcarbonat und rührte eine Stunde bei dieser Temperatur. Dann gab man tropfenweise 0,42 g (3,43 mmol) Butoxymethylchlorid hinzu und rührte 6 Stunden bei 80°C. Dann gab man weitere 0,11 g (0,87 mmol) Butoxymethylchlorid zu und rührte erneut 5 Stunden. Anschließend wurden 20 ml Wasser zugegeben, dreimal mit je 30 ml Methyl-tert.-butylether extrahiert, die vereinigten organischen Phasen zweimal mit je 20 ml Wasser gewaschen, mit Magnesiumsulfat getrocknet und unter vermindertem Druck eingeengt. Das Rohprodukt wurde flash-chromatographisch über eine Kieselgel-Säule gereinigt, Heptan/Ethylacetat (2/1).
Ausbeute: 0,66 g (68% der Theorie); Öl; C₂₀H₂₆N₄O₂ (MG = 370,7 g/mol) Massenspektrum: 371,4 (100%, M+H); 297,2 (33%);
¹H-NMR (DMSO-d₆, 200 MHz): δ = 0,82 (t, 3 H, (CH₂)₃CH₃); 1,48 (d, 6 H, CH(CH₃)₂); 1,14-1,56 (m, 4 H, CH₂(CH₂)₂CH₃); 3,50 (t, 2 H, OCH₂); 5,06 (m, 1 H, CH(CH₃)₂); 5,30 (s, 2 H, benzyl. H); 5,50 (s, 2 H, OCH₂N); 7,24-7,43 (m, 5 H, aromat. H); 8,31 (s, 1 H, N=CH)

### c) 1-Butoxymethyl-3-isopropylxanthin (Verbindung 42)

660 mg (1,78 mmol) des 1,3,7-trisubstituierten Xanthins aus Stufe b) wurden in 60 ml Ethanol über 86 mg Palladium (10%) auf Aktivkohle in 14 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/ Methanol (19/0,3).
Ausbeute: 416 mg (83% der Theorie); Schmelzpunkt: 131°C
C₁₃H₂₀N₄O₃ (MG = 280,3 g/mol); Massenspektrum: 281,2 (100%, M+H); 207,2 (30%)

### Beispiel 11: 1-Isobutoxymethyl-3-methylxanthin (Verbindung 48)

### a) 7-Benzyl-1-isobutoxymethyl-3-methylxanthin

Zu einer Suspension von 2,25 g (8,8 mmol) 7-Benzyl-3-methylxanthin (hergestellt gemäß Beispiel 1a) in 50 ml N-Methylpyrrolidon gab man bei 60°C 1,9 g (14,08 mmol) Kaliumcarbonat und rührte eine Stunde bei dieser Temperatur. Dann gab man tropfenweise 1,4 g (11,44 mmol) Isobutoxymethylchlorid hinzu und rührte 3 Stunden bei 80°C. Man gab weitere 0,5 g (4,4 mmol) Isobutoxymethylchlorid zu und rührte erneut 2 Stunden. Anschließend wurden 50 ml Wasser zugegeben und dreimal mit je 60 ml Methyl-tert.-butylether extrahiert. Die vereinigten organischen Phasen wurden zweimal mit je 30 ml Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (19/0,2).
Ausbeute: 2,54 g (85% der Theorie); Schmelzpunkt: 76°C
C₁₈H₂₂N₄O₃ (MG = 342,4 g/mol); Massenspektrum: 343,3 (100%, M+H); 269,2 (88%); 179.1 (24%)

### b) 1-Isobutoxymethyl-3-methylxanthin (Verbindung 48)

2,1 g (6,14 mmol) des 1,3,7-trisubstituierten Xanthins aus Stufe a) wurden in 50 ml Ethanol über 0,4 g Palladium (10%) auf Aktivkohle in 25 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/ Methanol (19/0,3).
Ausbeute: 0,59 g (38% der Theorie); Schmelzpunkt: 160°C
C₁₁H₁₆N₄O₃ (MG = 252,3 g/mol); Massenspektrum: 252 (7%, M); 196 (10%); 180 (100%); 179 (88%); 167 (56%)

### Beispiel 12: 1-sec-Butoxymethyl-3-ethylxanthin (Verbindung 52)

### a) 7-Benzyl-1-sec-butoxymethyl-3-ethylxanthin

Zu einer Suspension von 3,0 g (11,0 mmol) 7-Benzyl-3-ethylxanthin (hergestellt gemäß Beispiel 5 a) in 60 ml Dimethylformamid gab man bei 60°C 2,45 g (18,0 mmol) Kaliumcarbonat und rührte eine Stunde bei dieser Temperatur. Dann gab man tropfenweise 1,77 g (14,0 mmol) sec-Butoxymethylchlorid hinzu und rührte 5 Stunden bei 80°C. Man gab erneut 0,7 g (5,5 mmol) sec-Butoxymethylchlorid zu und rührte weitere 3 Stunden. Anschließend wurden 12 ml Wasser und 5 ml Methanol zugegeben und 2 Stunden bei 50°C gerührt. Dann wurden weitere 60 ml Wasser zugegeben, dreimal mit je 200 ml Methyl-tert.butylether extrahiert, die vereinigten organischen Phasen mit 200 ml Wasser gewaschen, mit Magnesiumsulfat getrocknet und unter vermindertem Druck eingeengt. Das Rohprodukt wurde flash-chromatographisch über eine Kieselgel-Säule gereinigt, Dichlormethan/Methanol (19,8/0,2),
Ausbeute: 3,29 g (84% der Theorie); Öl; C₁₉H₂₄N₄O₃ (MG = 356,4 g/mol) Massenspektrum: 356 (4%, M); 284 (71%); 271 (32%); 91 (100%);
¹H-NMR (DMSO-d₆, 200 MHz): δ = 0,73 (t, 3 H, CH₂CH₃); 1,05 (d, 3 H, CHCH₃); 1,21 (t, 3 H, NCH₂CH₃); 1,35 (quint., 2 H, CHCH₂CH₃); 3,57 (sixt., 1 H, CHCH₂); 4,02 (q, 2 H, NCH₂CH₃); 5,30 (AB-System, 2 H, OCH₂N); 5,50 (s, 2 H, benzyl. H); 7,23-7,40 (m, 5 H, aromat. H); 8,32 (s, 1 H, N=CH)

### b) 1-sec-Butoxymethyl-3-ethylxanthin (Verbindung 52)

2,73 g (7,66 mmol) des 1,3,7-trisubstituierten Xanthins aus Stufe a) wurden in 100 ml Ethanol über 273 mg Palladium (10%) auf Aktivkohle in 12,5 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Dichlormethan/Methanol (19,7/0,3).
Ausbeute: 1,82 g (89% der Theorie); Schmelzpunkt: 189°C
C₁₂H₁₈N₄O₃ (MG = 266,3 g/mol); Massenspektrum: 266 (4%, M); 194 (87%); 193 (100%); 181 (63%); 136 (87%)

### Beispiel 13: 1-(2-Methoxy-ethoxymethyl)-3-methylxanthin (Verbindung 53)

### a) 7-Benzyl-1 -(2-methoxy-ethoxymethyl)-3-methyl-xanthin

Das Gemisch aus 25,6 g (0,1 mol) 7-Benzyl-3-methylxanthin (hergestellt gemäß Beispiel 1a), 15,2 g (0,11 mol) Kaliumcarbonat und 16,2 g (0,13 mol) 2-Methoxy-ethoxymethyl-chlorid in 500 ml Acetonitril wurde 5 Stunden unter Rühren auf 50°C erwärmt, anschließend analog Beispiel 1b) aufgearbeitet und das gewonnene ölige Produkt mittels Filtration über eine Kieselgel-Säule im Fließmittel Chloroform/Methanol (10/1) gereinigt.
Ausbeute: 22,8 g (66,2% der Theorie); Öl; C₁₇H₂₀N₄O₄ (MG = 344,3 g/mol)

| | | | | |
|---|---|---|---|---|
| Analyse | Berechnet | C 59,29% | H 5,85% | N 16,27% |
| | Gefunden | C 59,01% | H 5,93% | N 16,02% |

### b) 1-(2-Methoxy-ethoxymethyl)-3-methylxanthin (Verbindung 53)

Die hydrogenolytische Debenzylierung von 22,7 g (0,066 mol) der Verbindung aus Stufe a) gemäß Beispiel 1c) lieferte nach chromatographischer Reinigung und Umkristallisation aus Ethanol 10,9 g Endprodukt (65% der Theorie).
C₁₀H₁₄N₄O₄ (MG = 254,3 g/mol); Schmelzpunkt: 188°C

| | | | | |
|---|---|---|---|---|
| Analyse | Berechnet | C 47,24% | H 5,55% | N 22,04% |
| | Gefunden | C 47,22% | H 5,45% | N 22,06% |

### Beispiel 14: 3-Ethyl-1-(2-(2-methoxyethoxy)-ethyl)-xanthin (Verbindung 56)

14 g (0,037 mol) 7-Benzyl-3-ethyl-1-(2-(2-methoxyethoxy)-ethyl)-xanthin wurden aus 7-Benzyl-3-ethylxanthin (hergestellt gemäß Beispiel 5a) und 1-Brom-2-(2-methoxyethoxy)-ethan (hergestellt gemäß Beispiel 2b) in einer Ausbeute von 98% der Theorie hergestellt (C₁₉H₂₄N₄O₄ (MG = 372,4 g/mol); Schmelzpunkt nach Umkristallisation aus Diisopropylether: 64°C;

| | | | | |
|---|---|---|---|---|
| Analyse | Berechnet | C 61,28% | H 6,50% | N 15,04% |
| | Gefunden | C 61,44% | H 6,49% | N 15,26%) |

und analog Beispiel 1c) hydrogenolytisch debenzyliert. Das erhaltene Rohprodukt wurde ohne säulenchromatographische Reinigung direkt aus Essigsäureethylester umkristallisiert. Ausbeute: 7,5 g (71,8% der Theorie); Schmelzpunkt: 140°C; C₁₂H₁₈N₄O₄ (MG = 282,3 g/mol)

| | | | | |
|---|---|---|---|---|
| Analyse | Berechnet | C 51,05% | H 6,43% | N 19,85% |
| | Gefunden | C 51,51% | H 6,37% | N 19,87% |

### Beispiel 15: 3-Methyl-1-(2-phenoxyethyl)-xanthin (Verbindung 60)

### a) 7-Benzyl-3-methyl-1-(2-phenoxyethyl)-xanthin

Zu einer Suspension von 3,0 g (11,7 mmol) 7-Benzyl-3-methylxanthin (hergestellt gemäß Beispiel 1 a) in 70 ml Dimethylformamid gab man bei 60°C 2,6 g (18,72 mmol) Kaliumcarbonat und rührte eine Stunde bei dieser Temperatur. Dann gab man tropfenweise 3,1 g (15,21 mmol) 2-Phenoxyethylbromid hinzu und rührte 5 Stunden bei 80°C. Anschließend wurde das Rohgemisch filtriert, das Filtrat unter vermindertem Druck eingeengt, in Dichlormethan aufgenommen, einmal mit 1 N Natronlauge und zweimal mit Wasser gewaschen. Die organischen Phasen wurden mit Magnesiumsulfat getrocknet und unter vermindertem Druck eingeengt. Das Rohprodukt wurde flash-chromatographisch über eine Kieselgel-Säule gereinigt, Heptan/ Ethylacetat (1/2).
Ausbeute: 3,52 g (80% der Theorie); Schmelzpunkt: 141°C; C₂₁H₂₀N₄O₃
(MG = 376,4 g/mol); Massenspektrum: 376 (2%, M); 283 (100%); 91 (87%)

### b) 3-Methyl-1-(2-phenoxyethyl)-xanthin (Verbindung 60)

3,0 g (8,0 mmol) des 1,3,7-trisubstituierten Xanthins aus Stufe a) wurden in 500 ml Ethanol über 0,3 g Palladium (10%) auf Aktivkohle in 6 Stunden hydriert. Man überlagerte mit Stickstoff, filtrierte den Katalysator ab und engte unter vermindertem Druck ein. Der Rückstand wurde flash-chromatographisch über eine Kieselgel-Säule gereinigt, Heptan/Ethylacetat (1/10).
Ausbeute: 1,09 g (48% der Theorie); Schmelzpunkt: 207°C; C₁₄H₁₄N₄O₃
(MG = 286,3 g/mol); Massenspektrum: 287,2 (45%, M+H); 193,1 (100%)

### Beispiel 16: 1-(4-Chlorphenoxymethyl)-3-methylxanthin (Verbindung 65)

### a) 7-Benzyl-1-(4-chlorphenoxymethyl)-3-methylxanthin

Zu einer Suspension von 3,0 g (12,0 mmol) 7-Benzyl-3-methylxanthin (hergestellt gemäß Beispiel 1 a) in 50 ml Dimethylformamid gab man bei 60°C 2,59 g (19,0 mmol) Kaliumcarbonat und rührte eine Stunde bei dieser Temperatur. Dann gab man tropfenweise 2,69 g (15,0 mmol) 4-Chlorphenoxymethylchlorid hinzu und rührte 8 Stunden bei 80°C. Anschließend wurde das Rohgemisch filtriert, das Filtrat unter vermindertem Druck eingeengt, in Dichlormethan aufgenommen, einmal mit 1 N Natronlauge und zweimal mit Wasser gewaschen. Die organischen Phasen wurden mit Magnesiumsulfat getrocknet und unter vermindertem Druck eingeengt. Das Rohprodukt wurde flash-chro-matographisch über eine Kieselgel-Säule gereinigt, Dichlormethan/Methanol (19,8/0,2). Ausbeute: 4,15 g (87% der Theorie); Schmelzpunkt: 96°C
C₂₀H₁₇ClN₄O₃ (MG = 396,8 g/mol); Massenspektrum: 398 (2%, ³⁷Cl, M); 396 (6%, ³⁵Cl, M); 269 (100%); 91 (72%)

### b) 1-(4-Chlorphenoxymethyl)-3-methylxanthin (Verbindung 65)

3,37 g (8,5 mmol) des 1,3,7-trisubstituierten Xanthins aus Stufe a) wurden in 450 ml Ethanol über 0,34 g Palladium (10%) auf Aktivkohle in 5 Stunden hydriert. Man überlagerte mit Stickstoff, filtrierte den Katalysator ab und engte unter vermindertem Druck ein. Der Rückstand wurde flash-chromatographisch über eine RP-18-Säule gereinigt, Wasser/Acetonitril (7/3).
Ausbeute: 0,91 g (34% der Theorie); Schmelzpunkt: 218°C
C₁₃H₁₁ClN₄O₃ (MG = 306,7 g/mol); Massenspektrum: 309,1 (6%, ³⁷Cl, M+H); 307,1 (19%, ³⁵Cl, M+H); 179,1 (100%); 167,1 (11%)

### Beispiel 17: 1-Benzyloxymethyl-3-methylxanthin (Verbindung 68)

### a) 3-Methyl-7-tritylxanthin

Zu einer Suspension von 3,9 g (23,5 mmol) 3-Methylxanthin in 85 ml Dimethylformamid gab man bei 60°C portionsweise 0,62 g (25,88 mmol) Natriumhydrid, rührte 1,5 Stunden bei dieser Temperatur und erhitzte auf 90°C. Dann gab man 6,6 g (23,67 mmol) Tritylchlorid in 30 ml Dimethylformamid zu und rührte 3 Stunden bei 90°C. Anschließend wurde heiß abgesaugt und unter vermindertem Druck eingeengt, der Rückstand wurde in 1 N Natronlauge aufgenommen, auf 80°C erwärmt und abgesaugt. Das Filtrat wurde mit 2 N Salzsäure auf pH 4-5 gebracht. Der dabei entstandene Niederschlag wurde flash-chromatographisch über eine Kieselgel-Säule gereinigt, Dichlormethan/Methanol (19/0,2). Ausbeute: 6,55 g (68% der Theorie); Schmelzpunkt: 242°C C₂₅H₂₀N₄O₂ (MG = 408,7 g/mol); Massenspektrum: 409,1 (21%, M+H); 244,2 (17%); 243,2 (100%); 167,0 (17%)

### b) 1-Benzyloxymethyl-3-methyl-7-tritylxanthin

Zu einer Lösung von 2,4 g (5,9 mmol) 3-Methyl-7-tritylxanthin aus Stufe a) in 50 ml Dimethylformamid gab man bei 60°C 1,3 g (9,44 mmol) Kaliumcarbonat und rührte eine Stunde bei dieser Temperatur. Dann gab man tropfenweise 1,06 ml (7,67 mmol) Benzyloxymethylchlorid hinzu und rührte 7 Stunden bei 80°C. Anschließend wurden 50 ml Wasser zugegeben und dreimal mit je 60 ml Methyl-tert.-butylether extrahiert. Die vereinigten organischen Phasen wurden zweimal mit je 30 ml Wasser gewaschen, mit Magnesiumsulfat getrocknet und unter vermindertem Druck eingeengt. Das Rohprodukt wurde flash-chromatographisch über eine Kieselgel-Säule gereinigt, Heptan/Ethylacetat (3/2).
Ausbeute: 1,57 g (51% der Theorie); Schmelzpunkt: 164°C; C₃₃H₂₈N₄O₃ (MG = 528,9 g/mol); Massenspektrum: 535,2 (74%, M + Li); 243,1 (100%)

### c) 1-Benzyloxymethyl-3-methylxanthin (Verbindung 68)

Zu einer Suspension von 1,2 g (2,27 mmol) 1,3,7-trisubstituiertes Xanthin aus Stufe b) in 11 ml Ethanol gab man eine Mischung von 1,1 ml Ethanol und 2,2 ml 1 N Salzsäure. Es wurde 1,5 Stunden unter Rückfluß gekocht, unter vermindertem Druck eingeengt und flash-chromatographisch über eine Kieselgel-Säule gereinigt, Dichlormethan/Methanol (19/0,5).
Ausbeute: 0,6 g (92% der Theorie); Schmelzpunkt: 208°C
C₁₄H₁₄N₄O₃ (MG = 286,3 g/mol); Massenspektrum: 287,2 (57%, M+H); 257,1 (77%); 179.1 (100%); 91,1 (24%)

### Beispiel 18: 1-(2-(4-Chlorbenzyloxy)-ethyl)-3-methylxanthin (Verbindung 70)

### a) 1-(2-(4-Chlorbenzyloxy)-ethyl)-3-methyl-7-tritylxanthin

Zu einer Lösung von 2,4 g (5,9 mmol) 3-Methyl-7-tritylxanthin (hergestellt gemäß Beispiel 17 a) in 50 ml N-Methylpyrrolidon gab man bei 60°C 1,3 g (9,44 mmol) Kaliumcarbonat und rührte eine Stunde bei dieser Temperatur. Dann gab man tropfenweise 1,57 g (7,67 mmol) 2-(4-Chlorbenzyloxy)-ethylchlorid hinzu und rührte eine Stunde bei 80°C. Dann gab man weitere 1,0 g (4,9 mmol) 2-(4-Chlorbenzyloxy)-ethylchlorid zu und rührte erneut eine Stunde. Anschließend wurden 50 ml Wasser zugegeben, dreimal mit je 60 ml Methyl-tert.-butylether extrahiert, die vereinigten organischen Phasen zweimal mit je 30 ml Wasser gewaschen, mit Magnesiumsulfat getrocknet und unter vermindertem Druck eingeengt. Das Rohprodukt wurde flash-chromatographisch über eine Kieselgel-Säule gereinigt, Heptan/Ethylacetat (3/2).
Ausbeute: 2,13 g (63% der Theorie); Schmelzpunkt: 179°C;
C₃₄H₂₉ClN₄O₃ (MG = 577,1 g/mol); Massenspektrum: 585 (5%, ³⁷Cl, M+Li), 583,2 (8%, ³⁵Cl, M + Li); 243,1 (100%)

### b) 1-(2-(4-Chlorbenzyloxy)-ethyl)-3-methylxanthin (Verbindung 70)

Zu einer Suspension von 1,3 g (2,26 mmol) 1,3,7-trisubstituiertes Xanthin aus Stufe a) in 14 ml Ethanol gab man eine Mischung von 1,4 ml Ethanol und 2,8 ml 1 N Salzsäure. Es wurde eine Stunde unter Rückfluß gekocht, unter vermindertem Druck eingeengt und flash-chromatographisch über eine Kieselgel-Säule gereinigt, Dichlormethan/Methanol (19/0,5).
Ausbeute: 0,72 g (95% der Theorie); Schmelzpunkt: 152°C
C₁₅H₁₅ClN₄O₃ (MG = 334,7 g/mol); Massenspektrum: 336 (1%, ³⁷Cl, M); 334 (2%, ³⁵Cl, M); 194 (100%); 179 (25%); 166 (65%)

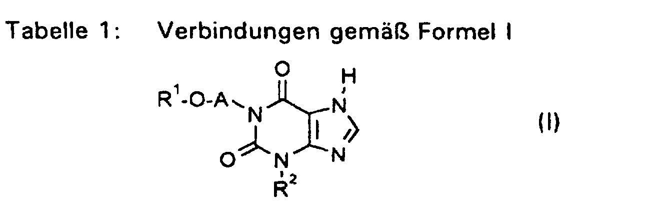

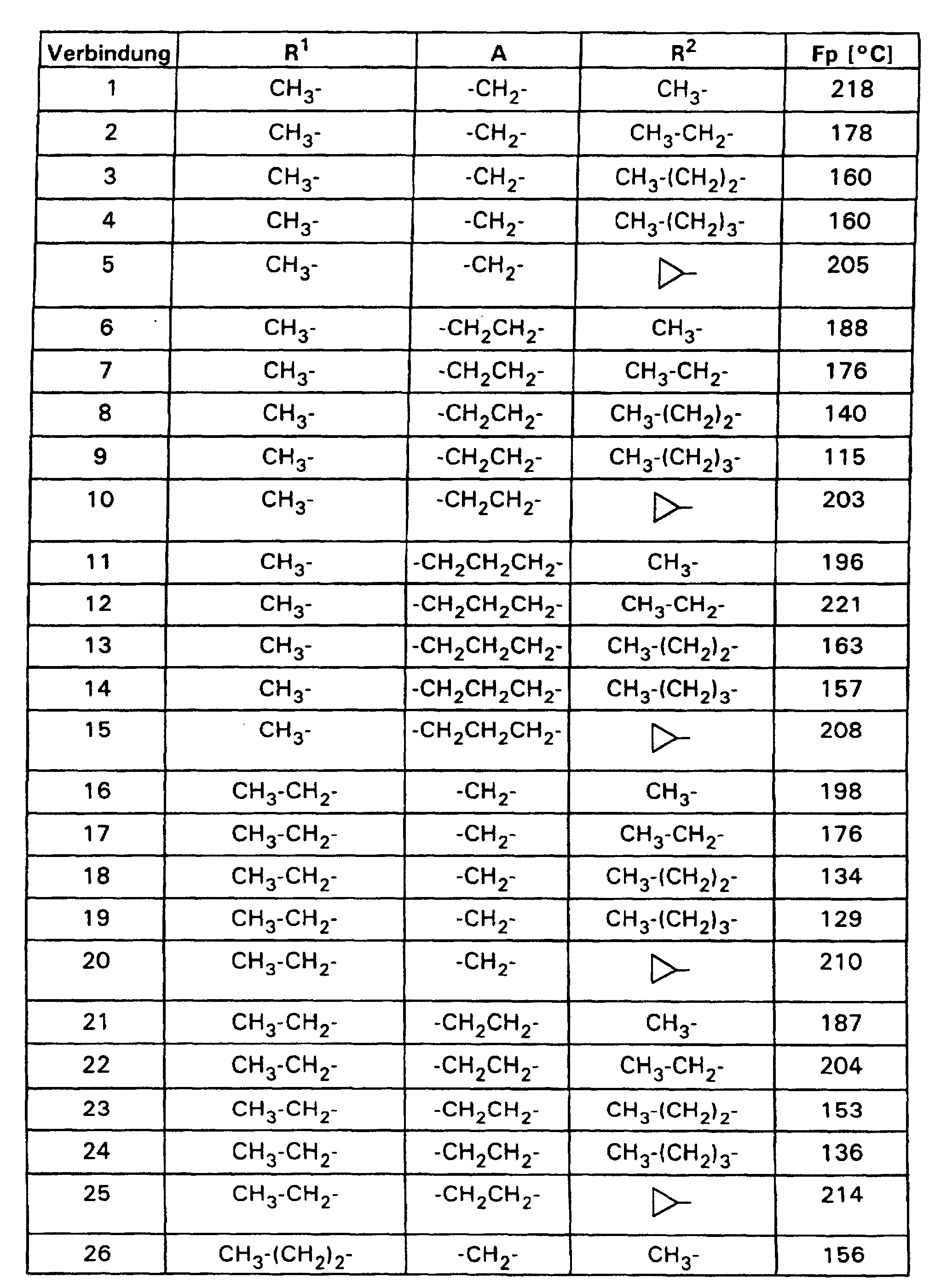

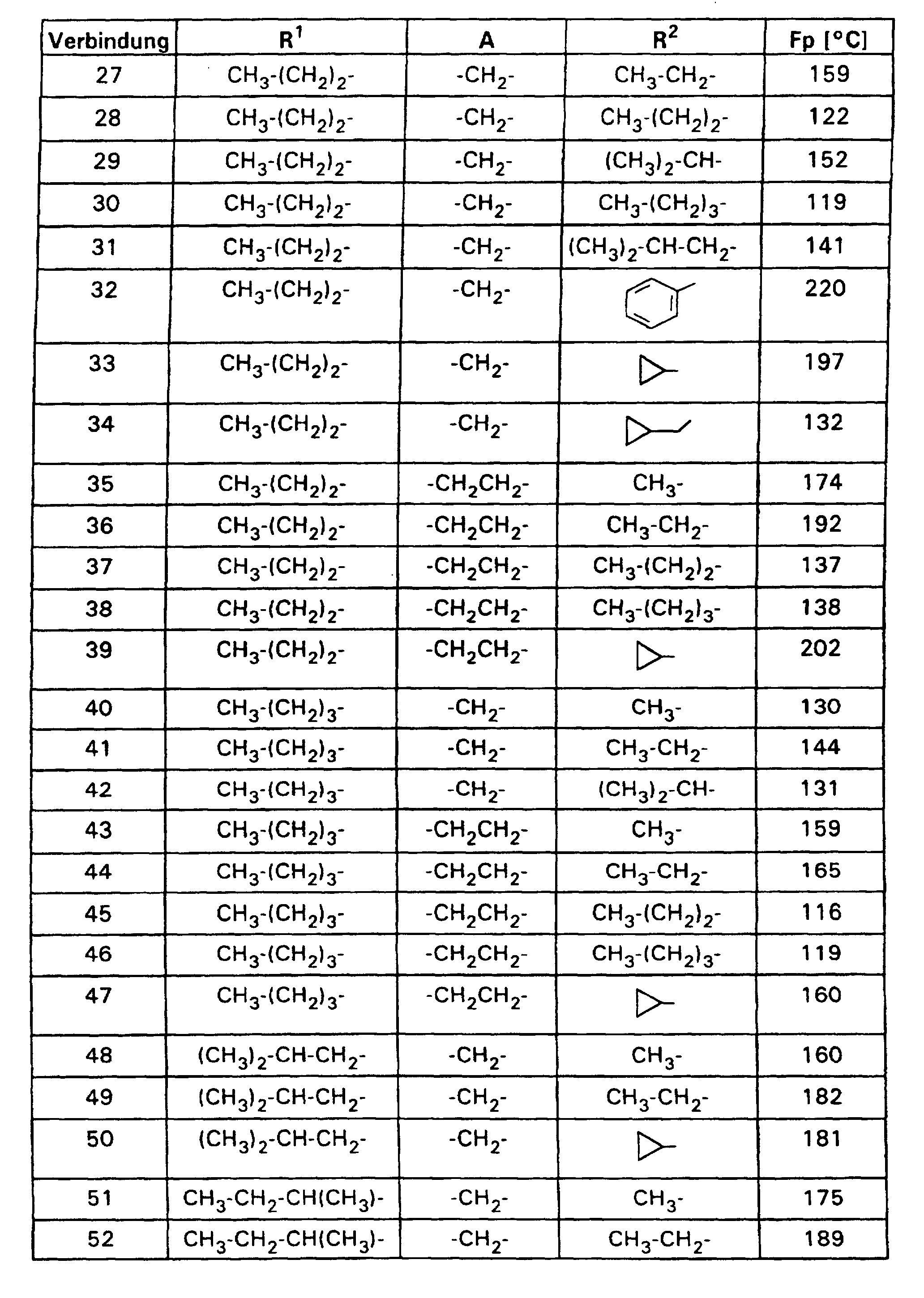

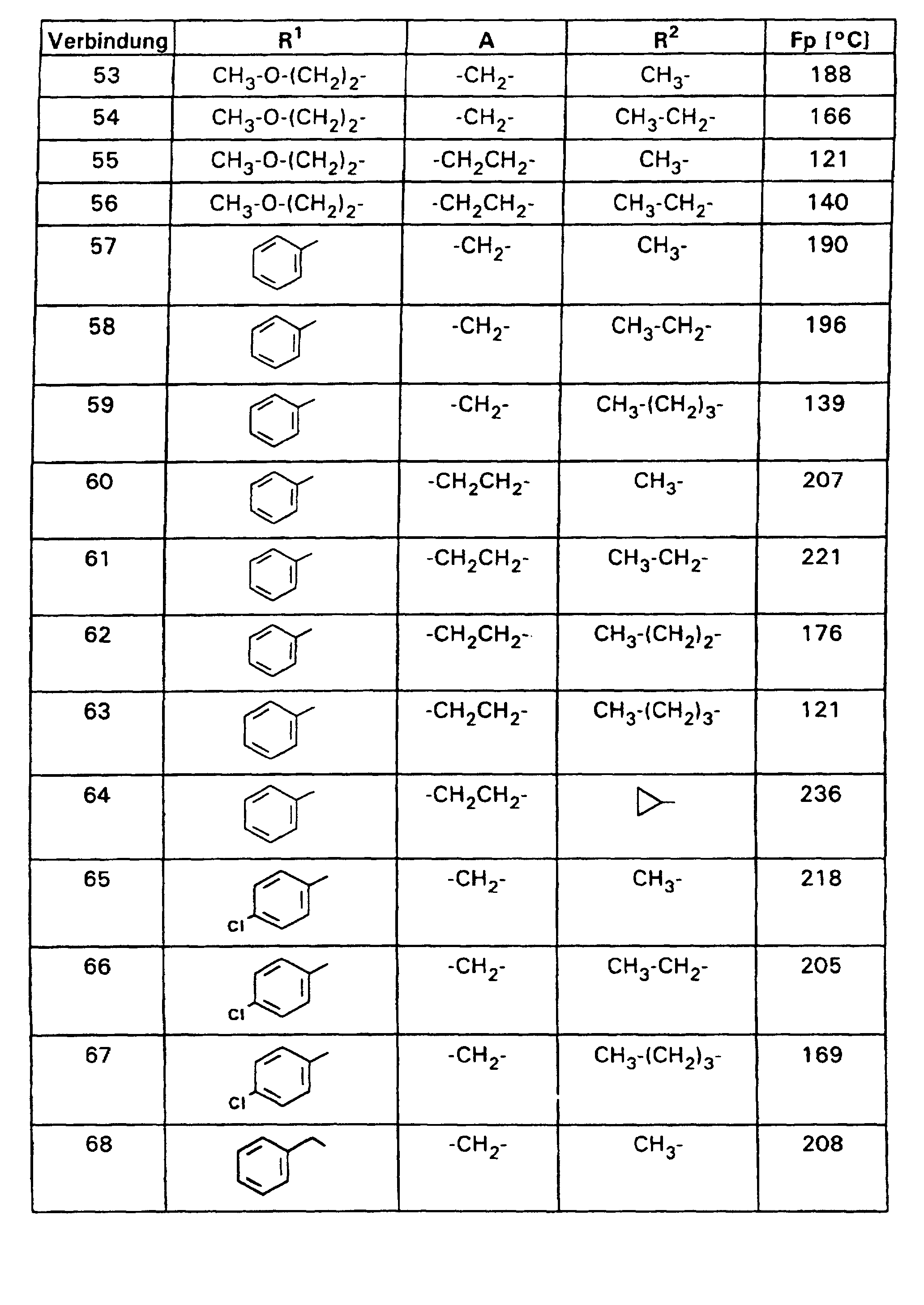

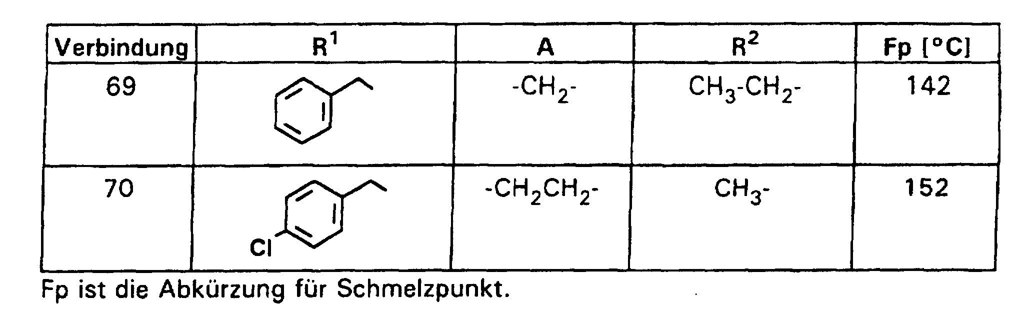

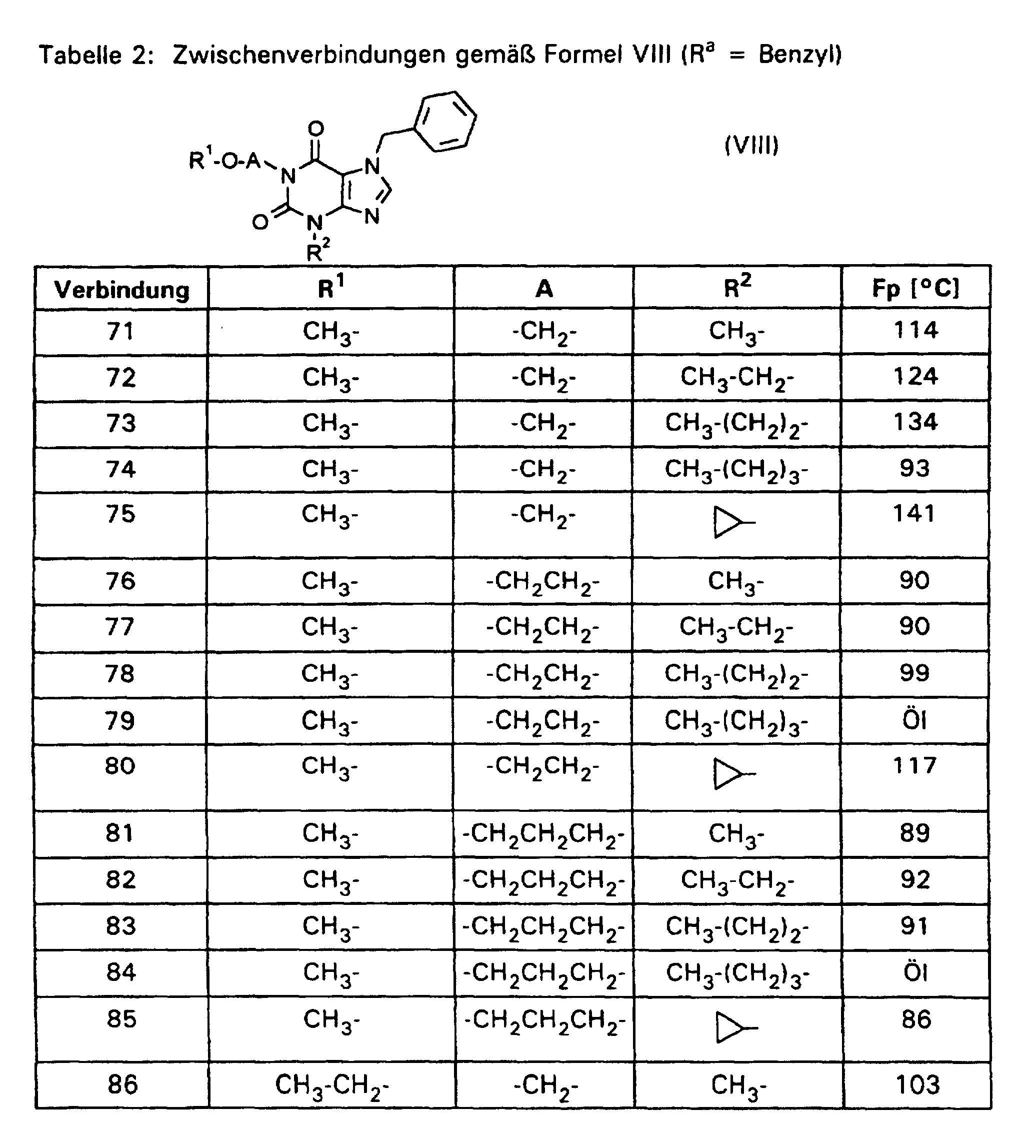

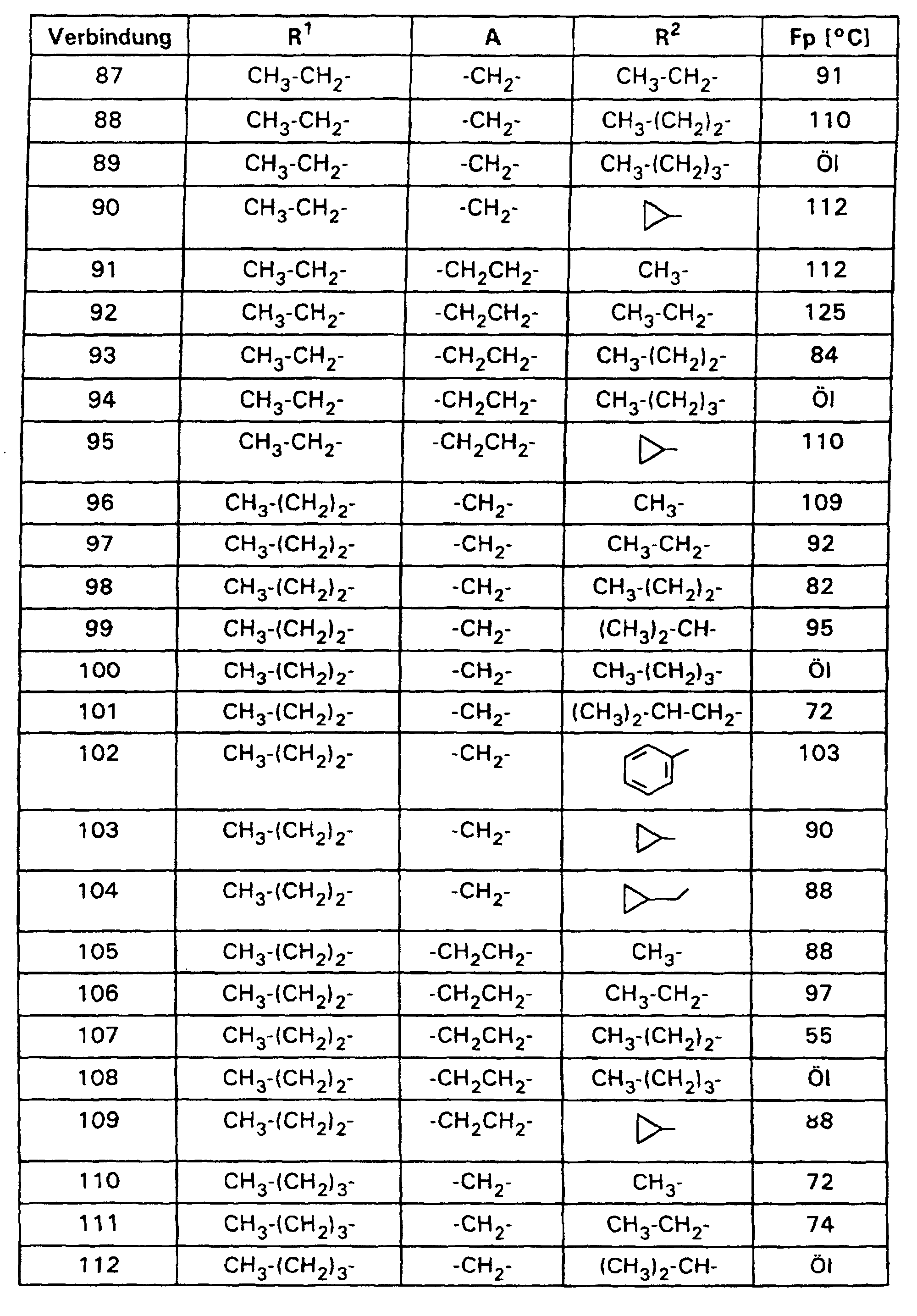

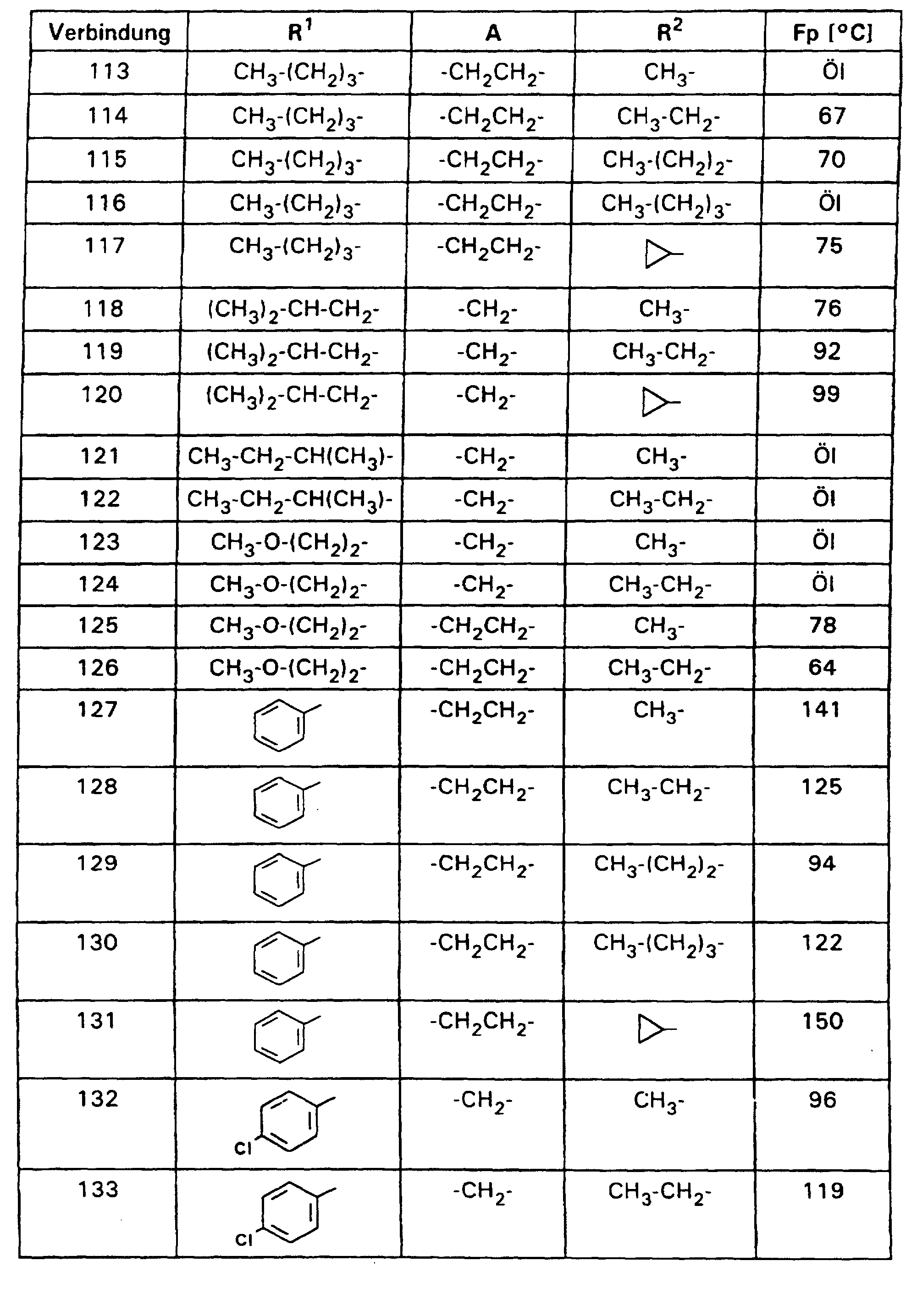

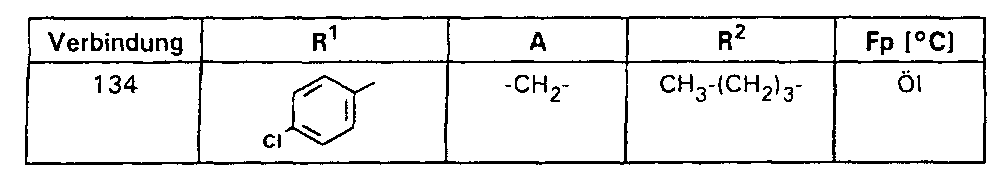

### Pharmakologische Prüfung und Ergebnisse

Die ausgeprägte Antischockwirkung der Verbindungen gemäß Formel I wurde in dem gut etablierten Modell des mit Endotoxin (LPS) induzierten tödlichen Schocks an C57BL/6-Mäusen anhand der Letalitätssenkung demonstriert.

Zur Durchführung der Versuche verabreichte man jedem Tier eine Mischung von 10 ng LPS aus Salmonella abortus equi und 7,5 mg Galaktosamin in 0,2 ml Phosphat-gepufferter, physiologischer Kochsalzlösung durch intravenöse Injektion, die in der Regel innerhalb von 6 bis 9 Stunden zum Tode führte. Die Prüfpräparate wurden eine Stunde vor der LPS-Provokation in einer Dosis von 100 mg/kg intraperitoneal appliziert. Die Tiere der Kontrollgruppe (n = 10) erhielten statt dessen reine 0,9%-ige Kochsalzlösung als Plazebo. Zur Beurteilung der Präparatwirkung wurde in dem behandelten Kollektiv (n = 10) die Anzahl der überlebenden Tiere 48 Stunden nach LPS-Gabe bestimmt und daraus unter Bezug auf die Mortalität in der Kontrollgruppe die prozentuale Hemmung der Letalität ermittelt. Die Versuchsergebnisse sind in Tabelle 3 wiedergegeben.

**Tabelle 3**

| Hemmung der LPS-induzierten Letalität an der Maus | |
|---|---|
| Verbindung aus Tabelle 1 | Hemmung der Letalität in % |
| 1 | 80 |
| 2 | 30 |
| 4 | 30 |
| 8 | 30 |
| 16 | 100 |
| 17 | 100 |
| 21 | 40 |
| 23 | 40 |
| 26 | 100 |
| 27 | 100 |
| 29 | 50 |
| 31 | 30 |
| 32 | 90 |
| 34 | 30 |
| 35 | 30 |
| 37 | 80 |
| 39 | 30 |
| 40 | 40 |
| 41 | 40 |
| 44 | 30 |
| 48 | 40 |
| 50 | 40 |
| 52 | 30 |
| 54 | 30 |

Im Rahmen eines breiteren pharmakologischen Screenings ließ sich außerdem zeigen, daß die Verbindungen der Formel I zusätzlich den ischämisch bedingten Zelluntergang im Zentralnervensystem nachhaltig zu hemmen vermögen. Sie eignen sich daher auch zur Behandlung und Prophylaxe von zerebrovaskulären Erkrankungen, wie Schlaganfall; transitorischen ischämischen Attacken (TIA); Multiinfarktdemenz; Demenz des gemischten Typs mit vaskulärer und degenerativer (Alzheimer) Komponente; Rückenmarkschädigungen; Hirntrauma infolge von Kopfverletzungen; und neuronalen Schäden nach Herzstillstand, (neonataler) Asphyxie und Reanimation sowie gefäßchirurgischen Eingriffen (z.B. Bypass-Operationen) im Bereich der das Gehirn versorgenden Hauptarterien.

Die neuronale Schutzwirkung der Theophylinderivate gemäß Formel I konnte unter anderem im Modell der transienten globalen Ischämie am Gerbil überzeugend nachgewiesen werden. Auch dieser Befund ist insofern überraschend, als Theophylin selbst unter vergleichbaren Versuchsbedingungen den ischämischen Nervenzellschaden weder bei Gerbil (J. Cereb. Blood Flow Metab. 1987, 7/1: 74 - 81) noch Ratte (J. Cereb. Blood Flow Metab. 1994, 14/1: 166 - 173) hemmt, sondern ihn vielmehr noch verstärkt.

Zur Versuchsdurchführung, die nach den Richtlinien des Deutschen Tierschutzgesetzes erfolgte, wurden 30 männliche Mongolische Gerbils mit einem Körpergewicht zwischen 60 und 70 g randomisiert auf zwei Kollektive mit jeweils 15 Tieren verteilt. Den Tieren des ersten Kollektives verabreichte man 30 Minuten nach der Ischämieperiode die jeweilige Testsubstanz durch intraperitoneale Injektion, während die Tiere des zweiten Kollektives, das als unbehandelte Kontrollgruppe diente, lediglich das gleiche Volumen des betreffenden Vehikels erhielten. Zur Erzeugung der temporären Vorderhirnischämie wurden die Tiere unter Halothan-Narkose auf einem beheizten Operationstisch in Rückenlage fixiert, beide Arteria carotides communes vorsichtig freigelegt und mittels Mikroaneurysma-Clips für drei Minuten verschlossen. 7 Tage nach der 3-minütigen Ischämieperiode wurden die Tiere in Halothan-Narkose dekapitiert, die Gehirne rasch und schonend entnommen, zunächst in Carnoy'scher Lösung (Ethanol/Chloroform/Essigsäure = 6/3/1) immersionsfixiert und dann in Paraffin eingebettet, anschließend 4 bis 6 µm dicke Coronarschnitte durch den Hippokampus etwa in Höhe des Bregma hergestellt und diese mit Hämatoxylin und Eosin gefärbt. Danach bestimmte man im Rahmen eines Blindversuches lichtmikroskopisch das Ausmaß der eosinophilen Nekrosen der Pyramidenzellen in der CA1-Region des Hippokampus anhand eines semiquantitativen histopathologischen Scores (0 = keine; 1 = leichte; 2 = mittelschwere; 3 = schwere und 4 = komplette Nekrosen). Als Bewertungsgröße für die neuroprotektive Wirkung diente die prozentuale Änderung des mittleren histopathologischen Scores der Präparatgruppe gegenüber dem der unbehandelten Kontrollgruppe. Die Versuchsergebnisse sind in Tabelle 4 zusammengestellt.

**Tabelle 4:**

| Hemmung des ischämischen Nervenzellschadens am Mongolischen Gerbil | | |
|---|---|---|
| Verbindung aus Tabelle 1 oder 2 | Dosis in mg/kg | Hemmung des neuronalen CA1-Hippokampusschadens in % |
| 5 | 10 | 20 |
| 6 | 10 | 20 |
| 7 | 10 | 33 |
| 17 | 10 | 66 |
| 24 | 10 | 30 |
| 25 | 10 | 24 |
| 27 | 10 | 67 |
| 27 | 5 | 40 |
| 28 | 10 | 30 |
| 30 | 10 | 24 |
| 36 | 10 | 23 |
| 39 | 10 | 36 |
| 44 | 10 | 64 |
| 54 | 10 | 36 |
| 58 | 10 | 20 |
| 61 | 10 | 23 |
| 63 | 10 | 27 |
| 66 | 10 | 23 |
| 77 | 10 | 33 |
| 82 | 10 | 30 |
| 103 | 10 | 32 |
| 114 | 10 | 27 |

## Patentansprüche

1. Verwendung von mindestens einer Verbindung der Formel I, 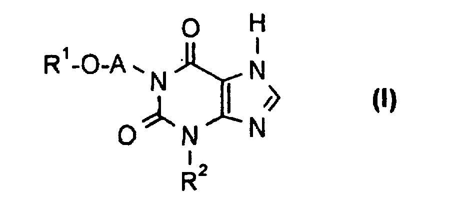 einschließlich ihrer gegebenenfalls stereoisomeren Formen und physiologisch verträglichen Salze, für die Herstellung eines Arzneimittels für die Behandlung und/oder Prophylaxe von Schockerkrankungen, wobei
R¹ für
a) geradkettiges oder verzweigtes (C₁-C₅)-Alkyl,
b) (C₁-C₂)-Alkoxy-(C₁-C₃)-alkyl oder
c) Phenyl oder Phenyl-(C₁-C₂)-alkyl steht, worin die Phenylreste unsubstituiert oder jeweils mit einem oder zwei Halogenatomen substituiert sind,
A für eine unverzweigte oder verzweigte (C₁-C₄)-Alkylenbrücke steht und
R² für
a) geradkettiges oder verzweigtes (C₁-C₅)-Alkyl,
b) (C₃-C₆)-Cycloalkyl,
c) (C₄-C₈)-Cycloalkyl-alkyl,
d) Phenyl oder
e) Phenyl-(C₁-C₂)-alkyl steht.

2. Verwendung gemäß Anspruch 1, **dadurch gekennzeichnet, daß** man mindestens eine Verbindung der Formel I gemäß Anspruch 1 einsetzt, wobei
R¹ für
a) geradkettiges oder verzweigtes (C₁-C₄)-Alkyl,
b) Methoxymethyl,
c) Methoxyethyl,
d) Phenyl,
e) 4-Chlorphenyl,
f) Benzyl oder
g) 4-Chlorbenzyl steht,
A für eine unverzweigte (C₁-C₃)-Alkylenbrücke steht und
R² für
a) geradkettiges oder verzweigtes (C₁-C₄)-Alkyl,
b) Cyclopropyl,
c) Cyclopropylmethyl,
d) Phenyl oder
e) Benzyl steht.

3. Verwendung gemäß der Ansprüche 1 oder 2, **dadurch gekennzeichnet, daß** man mindestens eine Verbindung der Formel I einsetzt, wobei
R¹ geradkettiges oder verzweigtes (C₁-C₄)-Alkyl,
A eine unverzweigte (C₁-C₃)-Alkylenbrücke und
R² geradkettiges oder verzweigtes (C₁-C₄)-Alkyl, Cyclopropyl oder Cyclopropylmethyl bedeuten.

4. Verwendung gemäß einem oder mehreren der Ansprüche 1 bis 3 für die Behandlung und/oder Prophylaxe von Systemic Inflammatory Response Syndrome, Sepsis, Sepsis-Syndrome, septischem Schock, Multiorganversagen, Acute Respiratory Distress Syndrome, hämorrhagischem oder traumatischem Schock, Verbrennungs- oder Dehydratations-Schock oder schockähnlichen Komplikationen beim Reperfusionssyndrom oder extrakorporalen Kreislauf.

5. Verwendung gemäß einem oder mehreren der Ansprüche 1 bis 4 zur Herstellung pharmazeutischer Zubereitungen für parenterale, orale, rektale, transdermale oder inhalative Verabreichung.

6. Verwendung nach Anspruch 5, **dadurch gekennzeichnet, daß** die hergestellte pharmazeutische Zubereitung zusätzlich eine effektive Menge mindestens eines Wirkstoffes, ausgewählt aus der Gruppe, bestehend aus Antikörpern gegen Entero- und Endotoxine (LPS), dem monozytären LPS-Rezeptor CD14, dem LPS-bindenden Protein LBP; Modulatoren des Zytokin-Netzwerkes; Hemmstoffen des Arachidonsäure-Stoffwechsels sowie der Koagulations- und Komplementkaskade; Antikoagulantien und Thrombozyten-Aggregationshemmem; Hemmem der Freisetzung und/oder der biologischen Wirkung von lytischen Enzymen; Sauerstoffradikalfängern; Schwermetall-Chelatoren; Inhibitoren der interzellulären Adhäsion und Antibiotika, enthält.

7. Verbindung der Formel I, deren gegebenenfalls stereoisomeren Formen und physiologisch verträglichen Salze, wobei
R¹ für
a) geradkettiges oder verzweigtes (C₁-C₅)-Alkyl,
b) (C₁-C₂)-Alkoxy-(C₁-C₃)-alkyl oder
c) Phenyl oder Phenyl-(C₁-C₂)-alkyl steht, worin die Phenylreste unsubstituiert oder jeweils mit einem oder zwei Halogenatomen substituiert sind,
A für eine unverzweigte oder verzweigte (C₁-C₄)-Alkylenbrücke steht und
R² für
a) geradkettiges oder verzweigtes (C₁-C₅)-Alkyl,
b) (C₃-C₆)-Cycloalkyl,
c) (C₄-C₈)-Cycloalkyl-alkyl,
d) Phenyl oder
e) Phenyl-(C₁-C₂)-alkyl steht,
wobei die Verbindungen der Formel I, worin a) R² für n-Propyl, R¹ für Methyl oder Ethyl und A für eine Ethylenbrücke oder b) R² für n-Propyl, R¹ für Methyl und A für eine n-Propylenbrücke oder c) R² für Methyl, R¹ für Ethyl und A für eine Methylenbrücke oder d) R² für Methyl, R¹ für Methyl und A für eine Ethylenbrücke stehen ausgenommen sind.

8. Verbindung der Formel I gemäß Anspruch 7, wobei
R¹ für
a) geradkettiges oder verzweigtes (C₁-C₄)-Alkyl,
b) Methoxymethyl,
c) Methoxyethyl,
d) Phenyl,
e) 4-Chlorphenyl,
f) Benzyl oder
g) 4-Chlorbenzyl steht,
A für eine unverzweigte (C₁-C₃)(C₁-C₃)-Alkylenbrücke steht und
R² für
a) geradkettiges oder verzweigtes (C₁-C₄)-Alkyl,
b) Cyclopropyl,
c) Cyclopropylmethyl,
d) Phenyl oder
e) Benzyl steht.

9. Verbindung der Formel I gemäß der Ansprüche 7 oder 8, wobei
R¹ geradkettiges oder verzweigtes (C₁-C₄)-Alkyl,
A eine unverzweigte (C₁-C₃)-Alkylenbrücke und
R² geradkettiges oder verzweigtes (C₁-C₄)-Alkyl, Cyclopropyl oder Cyclopropylmethyl bedeuten.

10. Verfahren zur Herstellung der Verbindung der Formel I gemäß den Ansprüchen 7 bis 9, **dadurch gekennzeichnet, daß** man
a) ein 3-substituiertes Xanthin der Formel II, 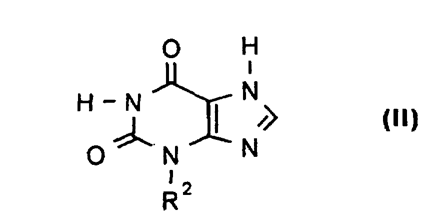 worin R² wie in Formel I definiert ist, ohne Kondensationsmittel oder in Gegenwart eines basischen Kondensationsmittels oder in Form seiner Salze mit einem Reagens der Formel III,
R^{a}-X (III)
worin R^{a} eine leicht eliminierbare Abgangsgruppe in Form der reduktiv oder auch hydrolytisch entfernbaren Benzyl-, Benzhydryl- oder Tritylgruppe mit unsubstituierten oder substituierten Phenylringen ist und X Chlor, Brom, Jod oder alternativ eine Sulfonsäureester- oder Phosphorsäureester-Gruppierung bedeutet, oder
b) ein 7-substituiertes Xanthin der Formel IV, 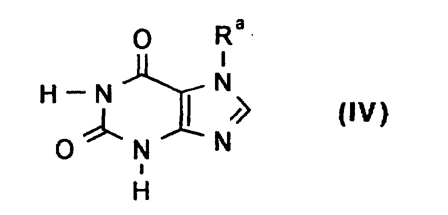 worin R^{a} Benzyl mit unsubstituiertem oder substituiertem Phenylring bedeutet, ohne Kondensationsmittel oder in Gegenwart eines basischen Kondensationsmittels oder in Form seiner Salze mit einem Reagenz der Formel V,
R²-X (V)
worin R² wie in Formel I und X wie in Formel III definiert sind, zu einem 3,7-disubstituierten Xanthin der Formel VI 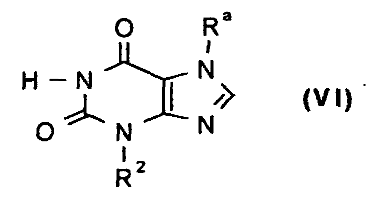 umsetzt, worin R² wie in Formel I und R^{a} wie in Formel III oder IV definiert sind, anschließend die Verbindung der Formel VI ohne Kondensationsmittel oder in Gegenwart eines basischen Kondensationsmittels oder in Form ihrer Salze mit einem Alkylierungsmittel der Formel VII,
R¹-O-A-X (VII)
worin R¹ und A wie in Formel I und X wie in Formel III definiert sind, in ein 1,3,7-trisubstituiertes Xanthin der Formel VIII, 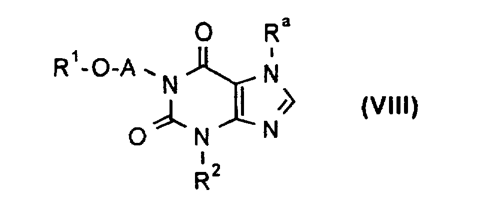 umwandelt, worin R¹, A und R² wie in Formel I und R^{a} wie in Formel III oder IV definiert sind,
und anschließend R^{a} aus dem Zwischenprodukt der Formel VIII unter Bildung der Verbindung der Formel I abspaltet und diese gegebenenfalls nach Trennung der stereoisomeren Formen, wahlfrei in ein physiologisch verträgliches Salz überführt.

11. Arzneimittel, **gekennzeichnet durch** den Gehalt einer therapeutisch effektiven Menge von mindestens einer Verbindung der Formel I gemäß einem oder mehreren der Ansprüche 7 bis 9 oder hergestellt nach Anspruch 10.

12. Verfahren zur Herstellung eines Arzneimittels nach Anspruch 11, **dadurch gekennzeichnet, daß** man mindestens eine Verbindung der Formel I gemäß, einem oder mehreren der Ansprüche 7 bis 9 mit pharmazeutisch akzeptablen und physiologisch verträglichen Träger- und Zusatzstoffen, Verdünnungsmitteln und/oder anderen Wirk- oder Hilfsstoffen in eine geeignete Darreichungsform bringt.

13. Verbindung der Formel VIII und/oder eine stereoisomere Form der Verbindung der Formel VIII, 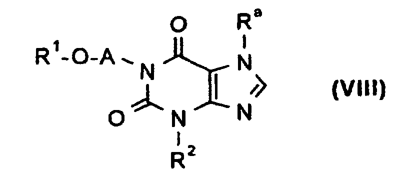 wobei R^{a} für Benzyl steht und R¹, A und R² wie in Formel I gemäß Anspruch 1 definiert sind,
wobei die Verbindung 7-Benzyl-1-ethoxymethyl-3-methylxanthin ausgenommen ist.

## Claims

1. The use of at least one compound of the formula I 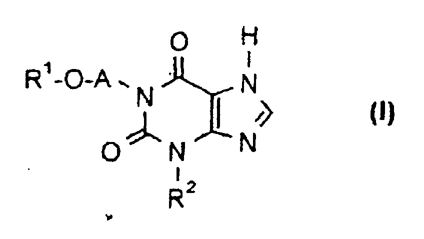 including its optionally stereoisomeric forms and physiologically tolerable salts, for the production of a pharmaceutical for the treatment and/or prophylaxis of shock disorders, where
R¹ is
a) straight-chain or branched (C₁-C₅)-alkyl,
b) (C₁-C₂)-alkoxy-(C₁-C₃)-alkyl or
or c) phenyl or phenyl-(C₁-C₂)-alkyl, in which the phenyl radicals are unsubstituted or each substituted by one or two halogen atoms,
A is an unbranched or branched (C₁-C₄)-alkylene bridge and
R² is
a) straight-chain or branched (C₁-C₅)-alkyl,
b) (C₃-C₆)-cycloalkyl,
c) (C₄-C₈)-cycloalkylalkyl,
d) phenyl or
e) phenyl-(C₁-C₂)-alkyl.

2. The use as claimed in claim 1, wherein at least one compound of the formula I as claimed in claim 1 is employed, where
R¹ is
a) straight-chain or branched (C₁-C₄)-alkyl,
b) methoxymethyl,
c) methoxyethyl,
d) phenyl,
e) 4-chlorophenyl,
f) benzyl or
g) 4-chlorobenzyl,
A is an unbranched (C₁-C₃)-alkylene bridge and
R² is
a) straight-chain or branched (C₁-C₄)-alkyl,
b) cyclopropyl,
c) cyclopropylmethyl,
d) phenyl or
e) benzyl.

3. The use as claimed in claims 1 or 2, wherein at least one compound of the formula I is employed, where
R¹ is straight-chain or branched (C₁-C₄)-alkyl,
A is an unbranched (C₁-C₃)-alkylene bridge and
R² is straight-chain or branched (C₁-C₄)-alkyl, cyclopropyl or cyclopropylmethyl.

4. The use as claimed in one or more of claims 1 to 3 for the treatment and/or prophylaxis of systemic inflammatory response syndrome, sepsis, sepsis syndrome, septic shock, multi-organ failure, acute respiratory distress syndrome; hemorrhagic or traumatic shock, burn or dehydration shock or shock-like complications in the reperfusion syndrome or extracorporeal circulation.

5. The use as claimed in one or more of claims 1 to 4 for the production of pharmaceutical preparations for parenteral, oral, rectal or transdermal administration or administration by inhalation.

6. The use as claimed in claim 5, wherein the pharmaceutical preparation prepared additionally contains an effective amount of at least one active compound, selected from the group consisting of antibodies against entero- or endotoxins (LPS), the monocytic LPS receptor CD14, the LPS-binding protein LBP; modulators of the cytokine network; inhibitors of arachidonic acid metabolism or of the coagulation and complement cascade; anticoagulants and platelet aggregation inhibitors; inhibitors of the release and/or the biological action of lytic enzymes; oxygen radical scavengers; heavy metal chelators; inhibitors of intercellular adhesion and antibiotics.

7. A compound of the formula I, its optionally stereoisomeric forms and physiologically tolerable salts, where
R¹ is
a) straight-chain or branched (C₁-C₅)-alkyl,
b) (C₁-C₂)-alkoxy-(C₁-C₃)-alkyl or
c) phenyl or phenyl-(C₁-C₂)-alkyl, in which the phenyl radicals are unsubstituted or each substituted by one or two halogen atoms,
A is an unbranched or branched (C₁-C₄)-alkylene bridge and
R² is
a) straight-chain or branched (C₁-C₅)-alkyl,
b) (C₃-C₆)-cycloalkyl,
c) (C₄-C₈)-cycloalkylalkyl,
d) phenyl or
e) phenyl-(C₁-C₂)-alkyl,
although compounds of the formula I in which a) R² is n-propyl, R¹ is methyl or ethyl and A is an ethylene bridge or b) R² is n-propyl, R¹ is methyl and A is an n-propylene bridge or c) R² is methyl, R¹ is ethyl and A is a methylene bridge or d) R² is methyl, R¹ is methyl and A is an ethylene bridge shall be excluded.

8. A compound of the formula I as claimed in claim 7, where
R¹ is
a) straight-chain or branched (C₁-C₄)-alkyl,
b) methoxymethyl,
c) methoxyethyl,
d) phenyl,
e) 4-chlorophenyl,
f) benzyl or
g) 4-chlorobenzyl,
A is an unbranched (C₁-C₃)-alkylene bridge and
R² is
a) straight-chain or branched (C₁-C₄)-alkyl,
b) cyclopropyl,
c) cyclopropylmethyl,
d) phenyl or
e) benzyl.

9. A compound of the formula I as claimed in claims 7 or 8, where
R¹ is straight-chain or branched (C₁-C₄)-alkyl,
A is an unbranched (C₁-C₃)-alkylene bridge and
R² is straight-chain or branched (C₁-C₄)-alkyl, cyclopropyl or cyclopropylmethyl.

10. A process for the preparation of the compound of the formula I as claimed in claims 7 to 9, which comprises
a) reacting a 3-substituted xanthine of the formula II 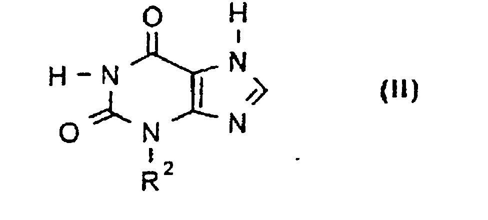 in which R² is as defined in formula I, without condensing agent or in the presence of a basic condensing agent or in the form of its salts with a reagent of the formula III
R^{a}-X (III)
in which R^{a} is an easily eliminable leaving group in the form of the reductively or alternatively hydrolytically removable benzyl, benzhydryl or trityl group having unsubstituted or substituted phenyl rings and X is chlorine, bromine, iodine or alternatively a sulfonic acid ester or phosphoric acid ester group, or
b) reacting a 7-substituted xanthine of the formula IV 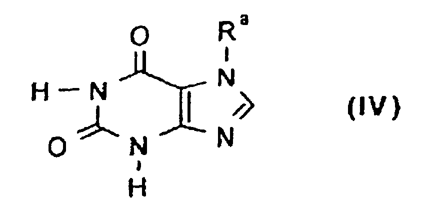 in which R^{a} is benzyl having an unsubstituted or substituted phenyl ring, without condensing agent or in the presence of a basic condensing agent or in the form of its salts with a reagent of the formula V
R²-X (V)
in which R² is as defined in formula I and X is as defined in formula III, to give a 3,7-disubstituted xanthine of the formula VI 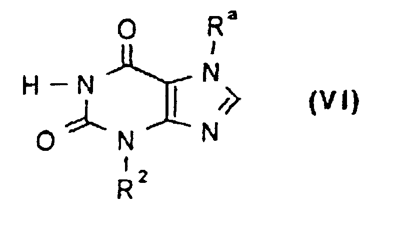 in which R² is as defined in formula I and R^{a} is as defined in formula III or IV, then converting the compound of the formula VI without condensing agent or in the presence of a basic condensing agent or in the form of its salts using an alkylating agent of the formula VII
R¹-O-A-X (VII)
in which R¹ and A are as defined in formula I and X is as defined in formula III, into a 1,3,7-trisubstituted xanthine of the formula VIII 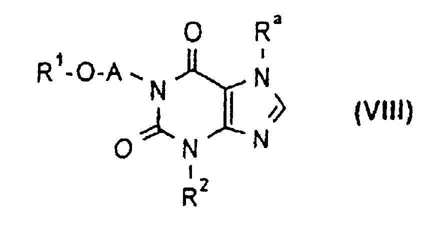 in which R¹, A and R² are as defined in formula I and R^{a} is as defined in formula III or IV,
and finally eliminating R^{a} from the intermediate of the formula VIII with formation of the compound of the formula I and optionally converting this, if appropriate after separation of the stereoisomeric forms, into a physiologically tolerable salt.

11. A pharmaceutical, which contains a therapeutically effective amount of at least one compound of the formula I as claimed in one or more of claims 7 to 9 or prepared as claimed in claim 10.

12. A process for the production of a pharmaceutical as claimed in claim 11, which comprises bringing at least one compound of the formula I as claimed in one or more of claims 7 to 9 into a suitable administration form using pharmaceutically acceptable and physiologically tolerable excipients and additives, diluents and/or other active compounds or auxiliaries.

13. A compound of the formula VIII and/or a stereoisomeric form of the compound of the formula VIII 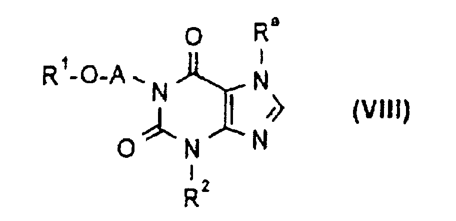 where R^{a} is benzyl and R¹, A and R² are as defined in formula I as in claim 1, although the compound 7-benzyl-1-ethoxymethyl-3-methylxanthine shall be excluded.

## Revendications

1. Utilisation d'au moins un composé de formule I 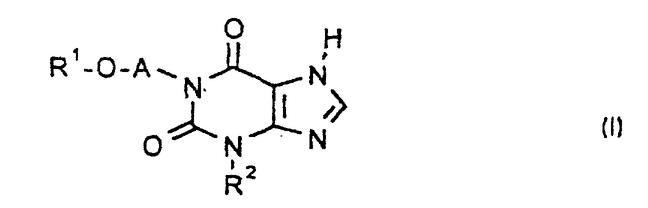 y compris leurs formes éventuellement isomères et des sels tolérés du point de vue physiologique, pour la préparation de médicaments pour le traitement et/ou la prévention de maladies de choc, où
R¹ représente des groupes
a) alkyle en C₁-C₅ à chaîne droite ou ramifiée,
b) (alkoxy en C₁-C₂)-alkyle en C₁-C₃ ou
c) phényle ou phénylalkyle en C₁-C₂, où les restes phényle sont non substitués ou substitués chacun par un ou deux atomes d'halogènes,
A représente
un pont alkylène en C₁-C₄ non ramifié ou ramifié et
R² représente des groupes
a) alkyle en C₁-C₅ à chaîne droite ou ramifiée,
b) cycloalkyle en C₃-C₆,
c) cycloalkylalkyle en C₄-C₈,
d) phényle ou
e) phénylalkyle en C₁-C₂.

2. Utilisation selon la revendication 1, **caractérisée en ce qu'**on utilise au moins un composé de formule I selon la revendication 1, où
R¹ représente des groupes
a) alkyle en C₁-C₄ à chaîne droite ou ramifiée,
b) méthoxyméthyle,
c) méthoxyéthyle,
d) phényle,
e) 4-chlorophényle,
f) benzyle ou
g) 4-chlorobenzyle,
A représente
un pont alkylène en C₁-C₃ non ramifié ou ramifié et
R² représente des groupes
a) alkyle en C₁-C₄ à chaîne droite ou ramifiée,
b) cyclopropyle,
c) cyclopropylméthyle,
d) phényle ou
e) benzyle.

3. Utilisation selon les revendications 1 ou 2, **caractérisée en ce qu'**on utilise au moins un composé de formule I, où
R¹ représente un groupe alkyle en C₁-C₄ à chaîne droite ou ramifiée,
A représente un pont alkylène en C₁-C₃ non ramifié et
R² représente des groupes alkyle en C₁-C₄ à chaîne droite ou ramifiée, cyclopropyle ou cyclopropylméthyle.

4. Utilisation selon une ou plusieurs des revendications 1 à 3 pour le traitement et/ou la prévention du syndrome de réponse systémique inflammatoire, de la septicémie, du syndrome de septicémie, du choc septique, de la défaillance multiorganes, du syndrome de détresse respiratoire aiguë, du choc hémorrhagique ou traumatique ou des complications similaires au choc dans le syndrome de réperfusion ou de la circulation extracorporelle.

5. Utilisation selon une ou plusieurs des revendications 1 à 4 pour la préparation de préparations pharmaceutiques pour l'administration par voies parentérale, orale, rectale, transdermique ou par inhalation.

6. Utilisation selon la revendication 5, **caractérisée en ce que** la préparation pharmaceutique préparée contient de plus une quantité efficace d'au moins un principe actif pris dans le groupe, comprenant des anticorps contre des entéro- et endotoxines (LPS), le récepteur de LPS CD14 monocytaire, la protéine de liaison de LP, la PLB ; les modulateurs du réseau de cytokines ; les inhibiteurs du métabolisme de l'acide arachidonique ainsi que de la cascade de coagulation et de la cascade de complément ; les anticoagulants et les inhibiteurs d'agrégation de thombocytes ; les inhibiteurs de la libération et/ou de l'action biologique d'enzymes lytiques ; les pièges à radicaux oxygénés ; les agents chélatants de métaux lourds ; les inhibiteurs de l'adhésion intercellulaire et les antibiotiques.

7. Composés de formule I, leurs formes éventuellement stéréoisomères et leurs sels toléré du point de vue physiologique, où
R¹ représente des groupes
a) alkyle en C₁-C₅ à chaîne droite ou ramifiée,
b) (alkoxy en C₁-C₂)-alkyle en C₁-C₃ ou
c) phényle ou phénylalkyle en C₁-C₂, où les restes phényle sont non substitués ou substitués chacun par un ou deux atomes d'halogènes,
A représente
un pont alkylène en C₁-C₄ non ramifié ou ramifié et
R² représente des groupes
a) alkyle en C₁-C₅ à chaîne droite ou ramifiée,
b) cycloalkyle en C₃-C₆,
c) cycloalkylalkyle en C₄-C₈,
d) phényle ou
e) phénylalkyle en C₁-C₂,
à l'exception des composés de formule I, où a) R² représente un groupe n-propyle, R¹ représente un groupe méthyle ou éthyle et A représente un pont éthylène ou ou b) R² représente un groupe n-propyle, R¹ représente un groupe méthyle et A représente un pont méthylène ou c) R² représente un groupe méthyle, R¹ représente un groupe éthyle et A représente un groupe propylène ou d) R² représente un groupe méthyle, R¹ représente un groupe méthyle et A représente un pont éthylène.

8. Composés de formule I selon la revendication 7, où
R¹ représente des groupes
a) alkyle en C₁-C₄ à chaîne droite ou ramifiée,
b) méthoxyméthyle,
c) méthoxyéthyle,
d) phényle,
e) 4-chlorophényle,
f) benzyle ou
g) 4-chlorobenzyle,
A représente
un pont alkylène en C₁-C₃ non ramifié ou ramifié et
R² représente des groupes
a) alkyle en C₁-C₄ à chaîne droite ou ramifiée,
b) cyclopropyle,
c) cyclopropylméthyle,
d) phényle ou
e) benzyle.

9. Composés de formule I selon les revendications 7 ou 8, où
R¹ représente un groupe alkyle en C₁-C₄ à chaîne droite ou ramifiée,
A représente un pont alkylène en C₁-C₃ non ramifié et
R² représente des groupes alkyle en C₁-C₄ à chaîne droite ou ramifiée, cyclopropyle ou cyclopropylméthyle.

10. Procédé pour la préparation des composés de formule I selon les revendications 7 à 9, **caractérisé en ce qu'**on fait réagir
a) une xanthine substituée en position 3 de formule II 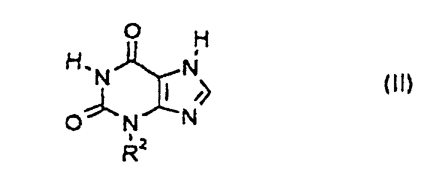 où R² est défini comme à la formule I, sans agent de condensation ou en présence d'un agent de condensation basique ou sous forme de ses sels sur un réactif de formule III
R^{a}-X (III)
où R^{a} représente un groupe partant, par exemple sous forme du groupe benzyle, benzhydryle ou trityle, facilement éliminable par réduction ou aussi par hydrolyse, par exemple avec des cycles phényle non substitués ou substitués, et X représente un atome de chlore, de brome ou d'iode ou alternativement un groupement d'ester d'acide sulfonique ou ester d'acide phosphorique, ou
b) une xanthine substituée en position 7 de formule IV 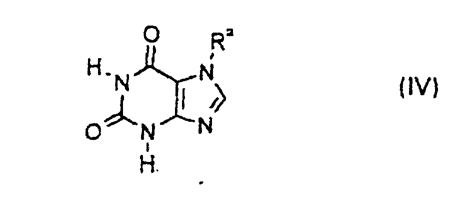 où R^{a} représente un groupe benzyle avec le reste phényle non substitué ou substitué, sans agent de condensation ou en présence d'un agent de condensation basique ou sous forme d'un sel sur un réactif de formule V
R²-X (V)
où R² est défini comme à la formule I et X est défini comme à la formule III,
pour obtenir une xanthine 3,7-disubstituée de formule VI 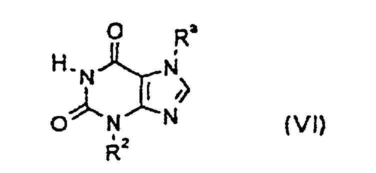 où R² est défini comme à la formule I et R^{a} est défini comme à la formule III ou IV, ensuite on transforme le composé de formule VI, sans agent de condensation ou en présence d'un agent de condensation basique ou sous forme de ses sels par un agent d'alkylation de formule VII
R¹-O-A-X (VII)
où R¹ et A sont définis comme à la formule III,
en une xanthine 1,3,7-trisubstituée de formule VIII 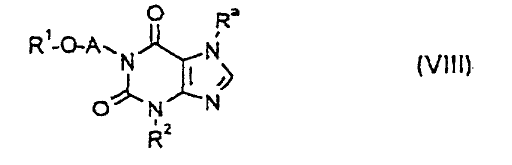 où R¹, A et R² sont définis comme à la formule I et R^{a} est défini comme à la formule III ou IV,
et ensuite on élimine le groupe R^{a} à partir du composé intermédiaire de formule VIII en formant le composé de formule I et en transformant celui-ci, éventuellement après séparation des formes stéréoisomères, au choix en un sel toléré du point de vue physiologique.

11. Médicament, **caractérisé par** une teneur thérapeutique efficace en au moins un composé de formule I selon une ou plusieurs des revendications 7 à 9 ou préparé selon la revendication 10.

12. Procédé pour la préparation d'un médicament selon la revendication 11, **caractérisé en ce qu'**on met en forme d'administration appropriée au moins un composé de formule I selon une ou plusieurs des revendications 7 à 9 avec des véhicules et additifs, des diluants et/ou autres principes actifs ou adjuvants acceptables en pharmacie et tolérés du point de vue physiologique.

13. Composé de formule VIII et/ou une forme stéréoisomère du composé de formule VIII, 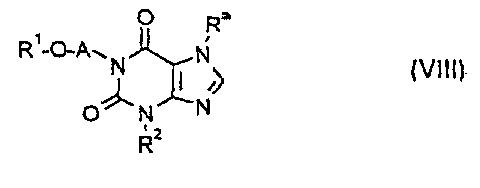 où
R^{a} représente un groupe benzyle et R¹, A et R² sont définis comme à la formule I selon la revendication 1,
le composé, la 7-benzyl-1-éthoxyméthyl-3-méthylxanthine, étant exclu.
